# EUROPEAN PATENT APPLICATION

(11) **EP 2 732 832 A2**
(43) Date of publication of application: **21.05.2014**
(21) Application number: 13192871.5
(22) Date of filing: 14.11.2013
(51) Int. Cl.: A61L 29/04, A61L 29/14, A61L 29/16, A61K 9/00, A61K 9/06

(54) **Drug delivery device comprising an active compound and a thermo-sensitive polymeric material**

(30) Priority: 14.11.2012 US 201261726093 P
(71) Applicant: Universitair Medisch Centrum Groningen (UMCG), 9713 GZ Groningen (NL)
(72) Inventor: van Asseldonk, Dirk Theodorus Andreas, 5612 AR Eindhoven (NL); van der Reijden, Guido, 5632 AG Eindhoven (NL); Abeln, Caroline Heleen, 5612 AR Eindhoven (NL); Hoogenboom, Richard, 4533 AB Terneuzen (NL); Busscher, Hendrik Jan, 5612 AR Eindhoven (NL)
(74) Representative: Nederlandsch Octrooibureau

(57) **Abstract**

The present invention relates to a drug delivery device comprising (a) an active compound; and (b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C; wherein the characteristics of diffusion or transport of the active compound can be modified in a reversible manner by supplying energy from an energy source.

The invention further relates to a method for releasing an active compound from a drug delivery device, the drug delivery device comprising (a) an active compound; and (b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C; wherein the active compound is released from the drug delivery device by reversibly modifying the transport characteristics of the thermo-sensitive polymeric material by supplying energy from an energy source.

## Description

### Technical field of the invention

The present invention relates to a drug delivery device, in particular a drug device for implantation in a mammalian body, comprising a thermo-sensitive polymeric material having a barrier effect against diffusion of active compounds, wherein the transport characteristics of said thermo-sensitive material can be modified by supplying energy from an energy source. In a particular embodiment, the invention relates to a catheter comprising the drug delivery device according to the invention.

The present invention also relates to a method for delivering a drug to a mammal in need thereof by employing a drug delivery device, wherein the delivery of the drug from the drug delivery device is actively controlled by means of supplying energy from an external energy source. The drug delivery device is suitable for implantation in a mammalian body, for incorporation into a prosthesis or an artificial organ.

### Background of the invention

It is known in the art that materials such as polymers can have a barrier effect against diffusion of an active compound. It is also generally known that these transport characteristics can be modified.

One problem in connection with known methods for modifying the abovementioned transport characteristics is that these are usually irreversible in some respect or other. A change in the physical state, including the degree of dissolution of a thermo-sensitive material, can result in the transport characteristics of a material being modified. Insofar as the material can again be brought into the original physical state, the active compound has diffused out. In the state of the art there is therefore a need for a drug delivery device and a method with which, when this is employed, the abovementioned transport characteristics can be modified in a reversible manner, so that the diffusion of an active compound can not only be interrupted in a controlled manner but can also, if desired, be repeated reproducibly.

Several systems are known from the prior art. US 6.312.708 discloses a system for controlled drug release of the neurotoxin botulinum toxin for a prolonged period of time. The system comprises a polymeric carrier comprising one or more biodegradable polymers and a stabilised botulinum toxin associated with the carrier. The system may be implanted subdermally, e.g. subcutaneously, intramuscularly and the like, or may be administered by injection. Preferably, the polymeric carrier is in the form of microspheres in which the botulinum toxin is incorporated, and the microspheres may be pressed into the form of a disc and implanted as a pellet. It is preferred that the polymeric carrier is comprised of non-toxic, non-immunological, biocompatible polymers such as poly(lactic acid), poly(glycolic acid) and the like. Such polymers are for example Medisorb® polymers which are said to be available from Medisorb Technologies International, now Alkermes, Inc. (cf. www.alkermes.com). Example 4 of US 6.312.708 discloses four sets of microspheres each differing in their rate of biodegradation that are compression moulded into a disc. The prolonged release of the botulinum toxin is therefore achieved by employing microspheres having a different rate of degradation in vivo. The polymers that may be used for the manufacture of the polymeric matrix can be selected from a wide group of materials (cf. US 6.312.708, column 20, lines 25 - 37), said materials having widely different properties such as glass transition temperature (Tg). For example, Alkermes, Inc. manufactures biodegradable polymers based on glycolic acid, lactide, caprolactone and mixtures thereof having glass transition temperatures within the range of -65° to 60° and melting points within the range of 60° to 230°C. The disadvantage of the system disclosed in US 6.312.708 is that it lacks controllability, i.e. that release of the drug cannot be initiated "at will" by some external activating source since it will inevitably release the drug in a continuous manner until all microspheres are degraded.

US 4.657.543 and US 4.779.806, incorporated by reference, disclose a process for delivering a biologically active substance, e.g. drugs such as steroids, anti-cancer drugs and infection suppressing agents, on demand wherein a polymeric matrix in which the biologically active substance is encapsulated is exposed to ultrasound. The frequency and intensity of the ultrasound energy employed is such that the polymeric matrix is degraded by ultrasound induced cavitation so that the drug is released. Suitable frequencies are about 20 kHz to about 1000 kHz and a suitable power is in the range of about 1W to about 30 W. It is worth noting that in this patent specification reference is made to power (because of the unit W) and not to intensity (unit W/cm²) as employed in the present application. This method has the disadvantage that due to the irreversible degradation of the polymeric matrix release of the biologically active substance is difficult to control. Another disadvantage of this method is that only a small increase in the rate of release could be achieved (about a factor 10).

US 4.767.402, incorporated by reference, discloses a transdermal patch comprising a protective cover, a matrix containing a drug, a support and an adhesive. The transdermal patch is applied to the skin and release of the drug through the skin is induced by ultrasound energy having a frequency of 20 kHz to 10 MHz and an intensity of 0 to 3 W/cm². Instead of a patch, an aqueous gel can be used in which the drug is suspended to enhance the efficiency of the ultrasound energy since it does not transmit well through air. Usually, drugs are systemically administered by transdermal patches and higher concentrations can only be achieved when the location of administration is just subcutaneous. In addition, many drugs cannot be administered by this method since they will not pass the skin. Furthermore, the method suffers from the disadvantage that release on demand is difficult to control since patches and in particular aqueous gels do not adhere sufficiently to the skin for a prolonged period of time. Hence, for a patient it would be required to replace the patch on a regular basis, e.g. every two days.

US 5.947.921 and US 6.041.253, incorporated by reference herein, disclose a method for transdermal delivery of a drug by means of a combination of ultrasound and a magnetic field, electroporation or ionthophoresis.

In US 5580573 a polymeric device for the controlled release of active agents contained in the device is disclosed. The device comprises a release rate controlling polymer. Said release rate controlling polymer in the device has a glass transition temperature greater than the normal storage or non-use temperature of the device. When heated to or above the glass transition temperature of said polymer, the active agent is released. The release of active agent can be switched on or off by the adjustment of the temperature of the polymeric device above or below the glass transition temperature of the polymer. Polymers having a glass transition temperature in the range of about 20 to about 50°C are preferred. Examples of suitable release rate controlling polymers include poly(vinyl acetate), polystyrene, poly(alkyl acrylate), poly(alkyl methacrylates), modified cellulosics, poly(vilyl pyrrolidone), aliphatic polyesters, and vinyl chloride polymers and their copolymers. In one embodiment, the drug delivery device constitutes of a combined monolithic-reservoir system comprising a matrix of a non-rate controlling polymer polymer having a low glass transition temperature such as C₂ - C₈ alkyl acrylate polymers and their copolymers, poly(vinyl alkyl ethers), polyisobutylene and polysiloxanes, and a rate controlling polymer as was described above.

In WO 2004/113422 a drug delivery device is disclosed, said device comprising a thermo-sensitive polymeric material having a barrier effect against diffusion of chemical substances or against electron transport, in particular an active compound, and having a glass transition temperature within the range of about 0° to about 90°C, and an active compound, wherein the characteristics of diffusion or transport of the active compound can be modified in a reversible manner by supplying energy from an energy source. Preferably, a poly(lactic acid-co-glycolic acid), polymethyl methacrylate, poly(ethylene-co-vinyl acetate) or a nylon is used as the thermo-sensitive polymeric material. More preferably, the thermo-sensitive polymeric material comprises a polymer or a copolymer comprising a monomer selected from the group consisting of a hydroxyl alkanoate wherein the alkyl group comprises 1 to 12 carbon atoms, lactide, glycolide, ε-caprolactone, 1,4-dioxane-2-one, 1,5-dioxepan-2-one, trimethylene carbonate (1,3-dioxane-2-one) and mixtures thereof. Even more preferred thermo-sensitive polymeric materials include (meth)acrylic (co)polymers, polyester urethanes, polyester amides, polyether esters such as polyethylene glycol terephtalate-polybutylene terephalate (PEGT/PBT), polyethylene glycol and the natural polymers such as polyhydralonic acid, iso-sorbide, dextran, collagens and mixtures thereof. Another even more preferred thermo-sensitive polymeric material is polybutyl methacrylate.

In WO 2008/016297, a drug delivery device of which the release characteristics are modified on demand by supplying infrared irradiation is disclosed, said device comprising a thermo-sensitive polymeric material, a pharmaceutically or biologically active component and an infrared absorbing compound. Said thermo-sensitive polymeric material preferably comprises poly(lactic acid-co-glycolic acid), polymethyl (meth)acrylate, poly(N-isopropylacrylamide), poly(N,N-dimethylacrylamide), a nylon, or a polymer or a copolymer comprising a monomer selected from the group consisting of a hydroxyl alkanoate wherein the alkyl group comprises 1 to 12 carbon atoms, lactide, glycolide, ε-caprolactone, 1,4-dioxane-2-one, 1,5-dioxepan-2-one, trimethylene carbonate (1,3-dioxane-2-one) and mixtures thereof, as well as polymers having side-chain crystallinity. More preferred thermo-sensitive polymeric materials include (meth)acrylic (co)polymers, polyester urethanes, polyester amides, polyether esters such as polyethylene glycol terephtalate - polybutylene terephalate (PEGT/PBT), polyethylene glycol and the natural polymers such as poly(hydralonic acid), iso-sorbide, dextran, collagens and mixtures thereof. Even more preferred thermo-sensitive polymeric materials include poly(n-butyl methacrylate) and poly(n-butylmethacrylate-co-methylmethacrylate).

A disadvantage of several drug delivery devices known in the art is that the permeability for an active compound of the polymeric material used in the device is often insufficient, leading to insufficient release of the active compound. Alternatively, in other drug delivery devices known in the art, the transport properties of the active compound within the polymeric material used in the drug delivery device are very high, leading to release of the active compound in a short timeframe. These drug delivery devices are therefore generally unsuitable for a repeated release of an active compound, over a longer period of time. In general, the barrier effect against diffusion of an active compound of polymers used in drug delivery devices known in the art is either too low or too high. In addition, polymeric materials used in drug delivery materials known in the art are often not sufficiently biocompatible.

Biomaterials are indispensable for restoration of function due to e.g. wear due to increased average life-expectancy, oncological surgery and trauma. In addition biomaterials, like catheters, are used as temporary devices for treatment during hospitalization. The unfavourable surface properties of nearly all biomaterials applied are such that they attract microorganisms to form so called biofilms which induce Biomaterial Associated Infections (BAI), occurring across all implants.^{1,2} The consequences of BAI are enormous: a patient with an infected implant starts a series of hospital visits and antibiotic use, with the almost certain outcome that the infected implant will have to be replaced at high costs and great discomfort for the patient. Unsuccessful treatment may eventually lead to loss of limbs or even death³, whereas infectious complications with central venous catheters poses a clinical dilemma: its removal to cure infection will disrupt the chemotherapy, while maintaining the catheter may yield the risk of death due to sepsis of an already weakened patient.

Antimicrobial therapies often do not sufficiently cure BAI because biofilms appear to form very effective barriers to anti-microbials, attributed to the low biofilm permeability for nutrients and antibiotics.⁴⁻⁶ Also because antibiotic prophylaxis may give rise to increasing bacterial resistance⁷, major efforts are put into the prevention of these type of infections by the development of anti-microbial implant coatings. Various coating strategies have been identified which apply to different origins of infection.

Effective in preventing infection due to per-operative contamination (primary surgery) are coatings which locally release antimicrobials during a short time. In order to prevent infections due to contaminated tissue in revision surgery or catheter infections during medium to long hospitalisation periods, long lasting functionality is needed (1 - 10 days for revision operations and up to 2 months for catheter applications). Current antimicrobial release systems on the market show a concentration driven release. As a result release rates are slowly decreasing, which results in less efficacy over longer time periods than a week. On-demand release coatings, which could extend the release to periods longer than a month, would therefore be more appropriate. Additionally, on demand delivery may create the possibility to dramatically reduce the total amount of active agent that is administered during use of the device and is therefore thought to avoid the development of anti-microbial resistance.

### Summary of the invention

The present invention relates to a drug delivery device, in particular for implantation in a mammalian body, said drug device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the characteristics of diffusion or transport of the active compound can be modified in a reversible manner by supplying energy from an energy source.

In a preferred embodiment of the drug delivery device according to the invention, the drug delivery device comprises:
(i) a central region comprising said active compound (a) and said thermo-sensitive polymeric material (b), and
(ii) an enveloping material surrounding said central region, said enveloping material being thermally insulating and permeable for the active compound.

The present invention particularly relates to a catheter comprising a drug delivery device according to the invention comprising:
(i) a central region comprising said active compound (a) and said thermo-sensitive polymeric material (b), and
(ii) an enveloping material surrounding said central region, said enveloping material being thermally insulating and permeable for the active compound.

The present invention also relates to a method for releasing an active compound from a drug delivery device according to the invention, the drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the active compound is released from the drug delivery device by reversibly modifying the transport characteristics of the thermo-sensitive polymeric material by supplying energy from an energy source.

The present invention provides a drug delivery system that does not have the disadvantages of the prior art drug delivery systems.

One of the advantages of the drug delivery device and the method for releasing an active compound from a drug delivery device according to the invention is that the thermo-sensitive material comprising a copolymer of styrene and C₁-C₁₂ alkyl (meth)acrylate has advantageous characteristics as compared to thermo-sensitive polymers used in drug delivery devices known in the art. Said thermo-sensitive material comprising a copolymer of styrene and C₁-C₁₂ alkyl (meth)acrylate has for example an enhanced diffusion of active compound when heated above its glass transition temperature Tg, as well as an improved barrier effect against diffusion of an active compound at temperatures below its Tg.

### Brief description of the figures

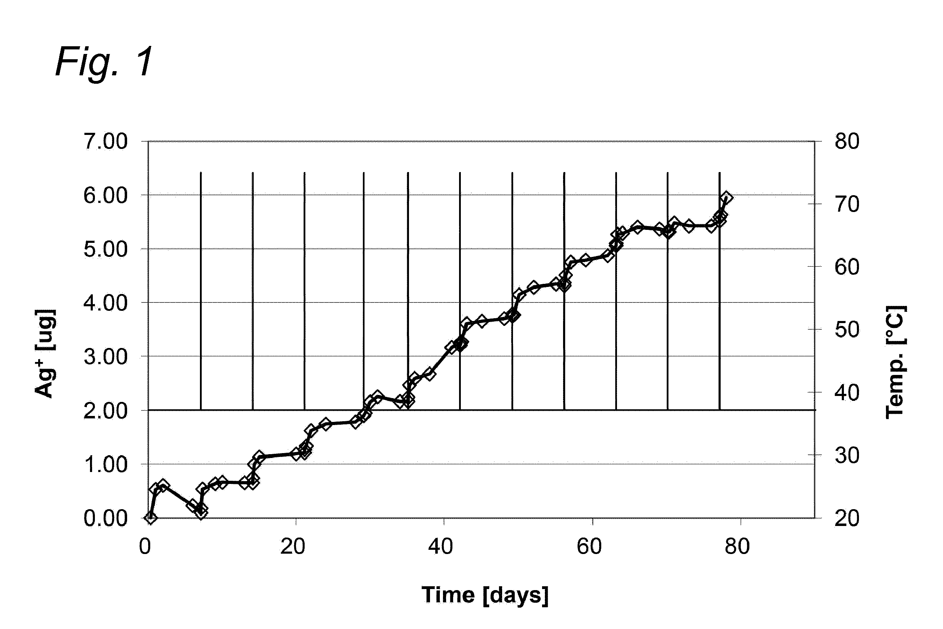

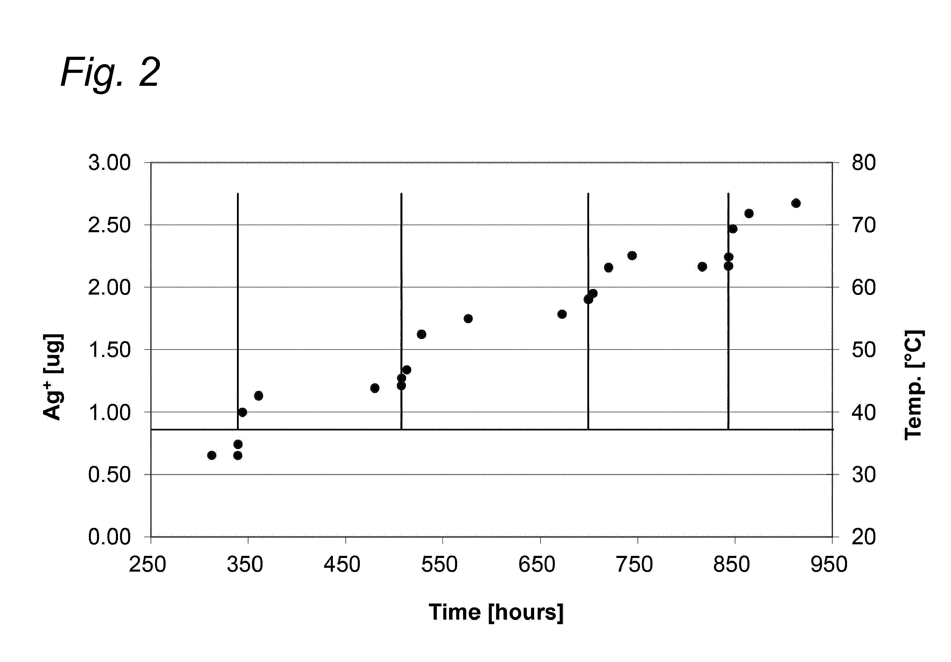

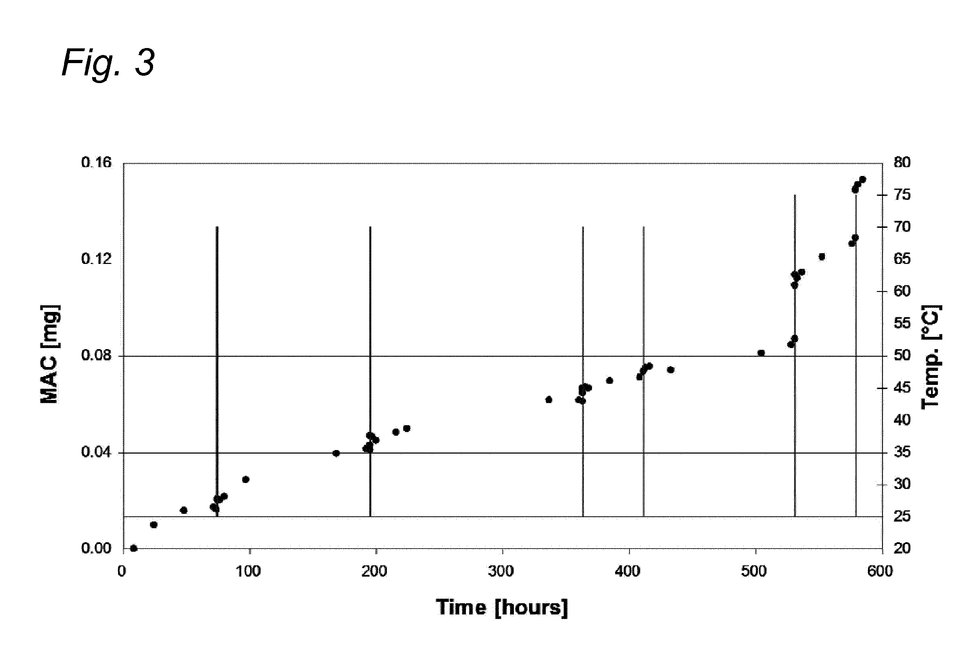

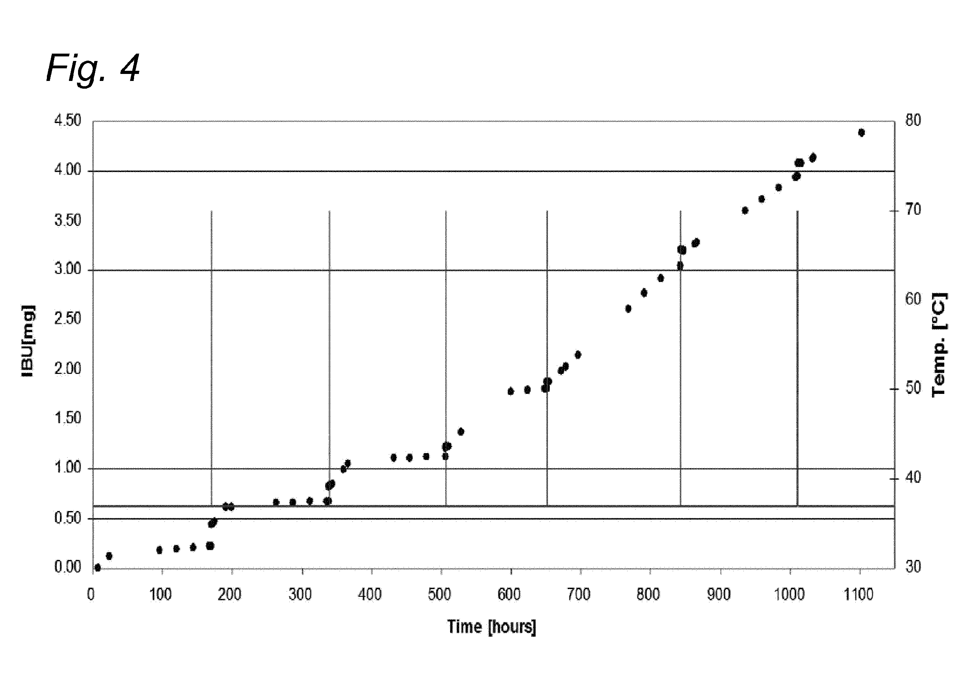

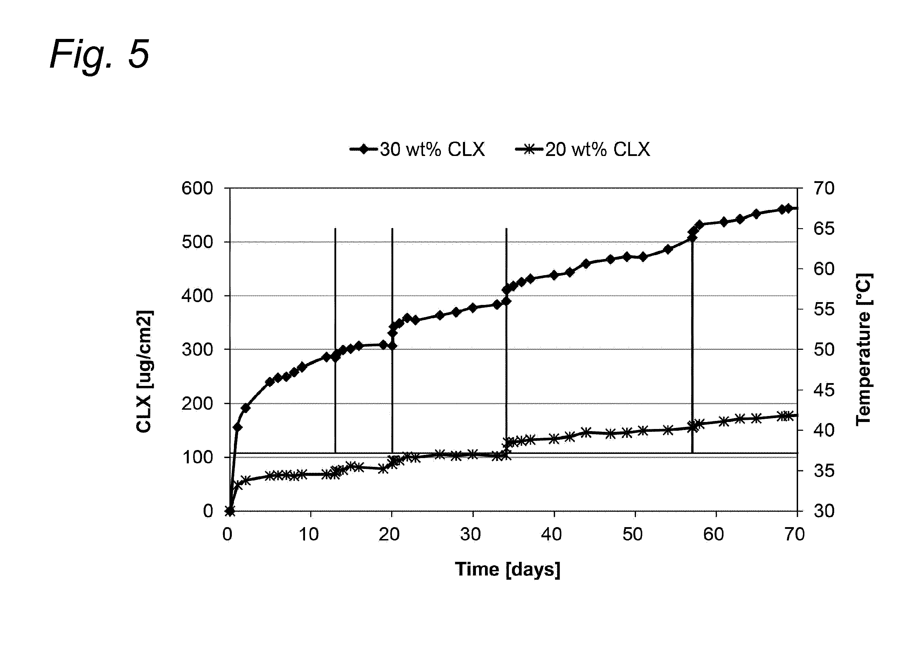

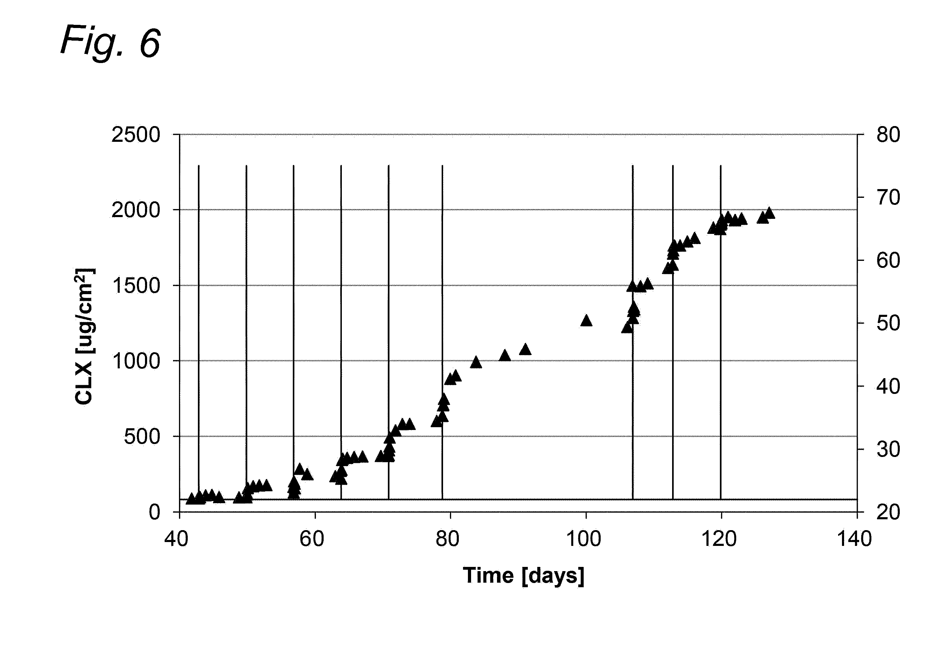

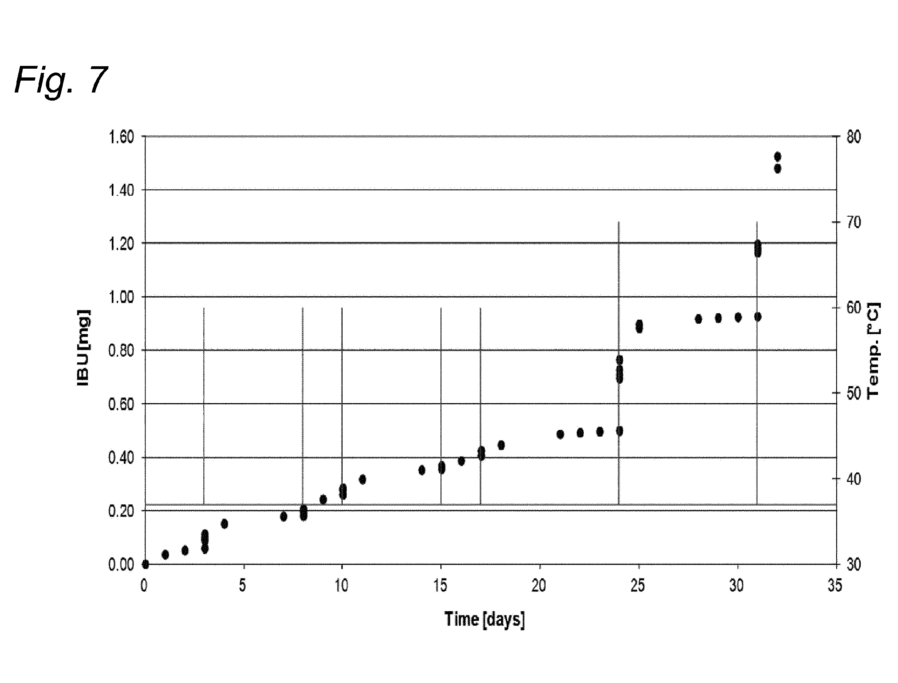

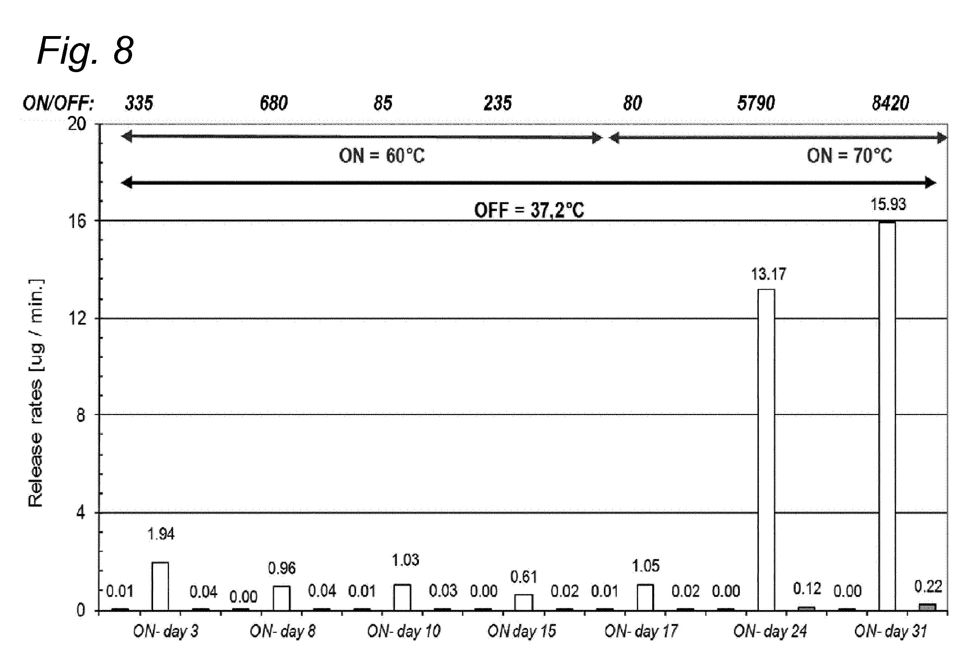

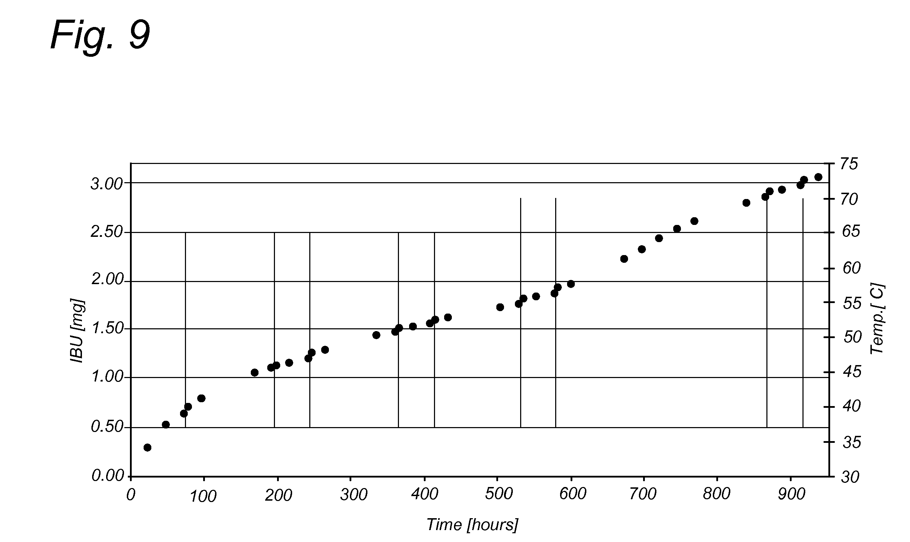

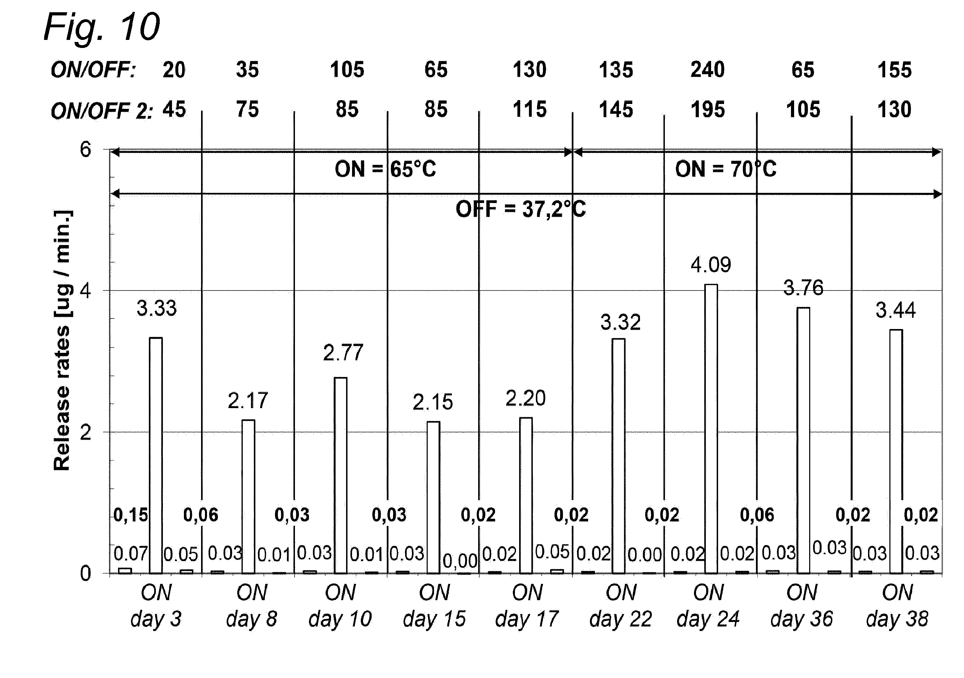

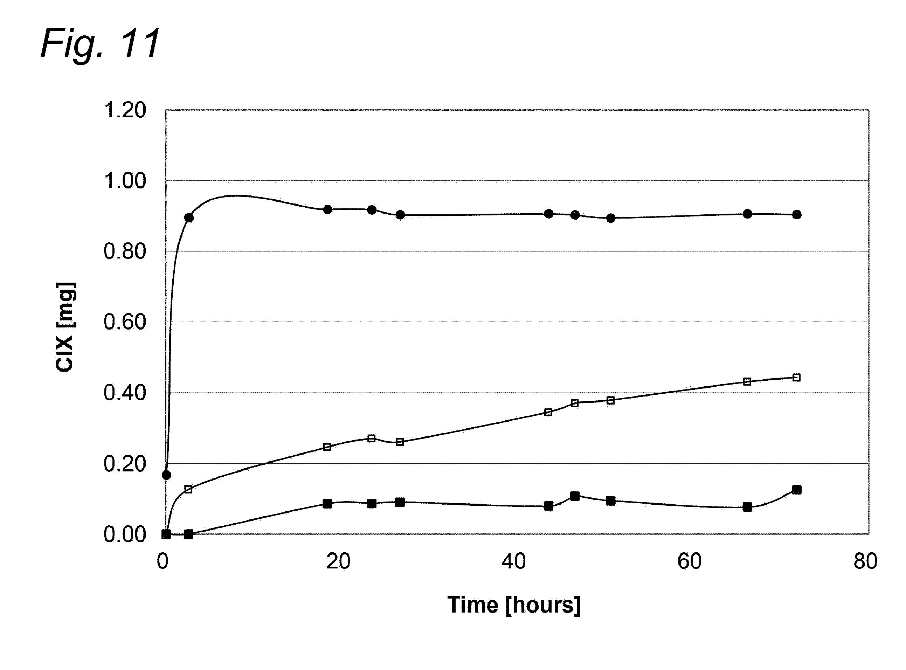

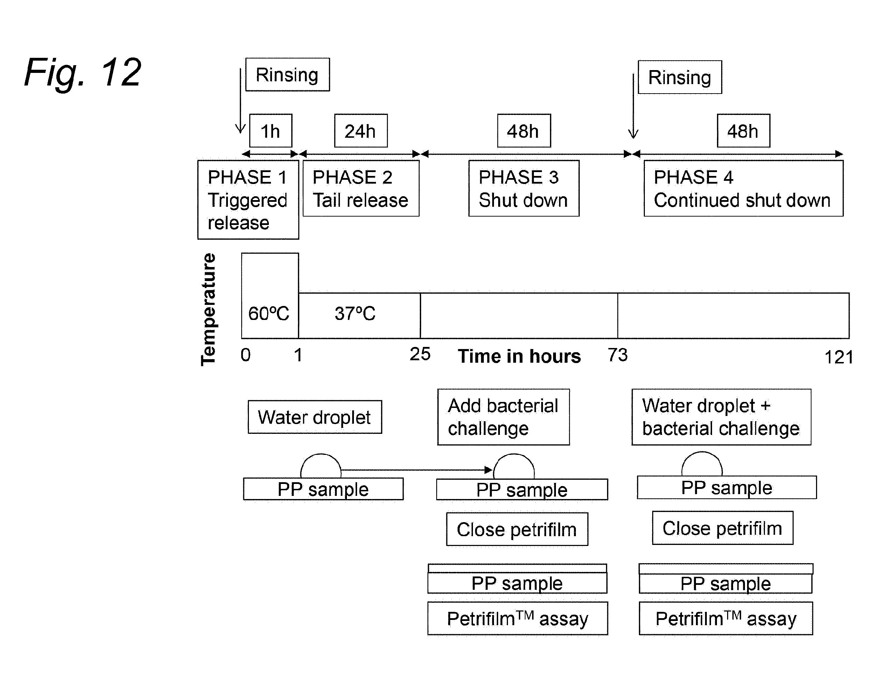

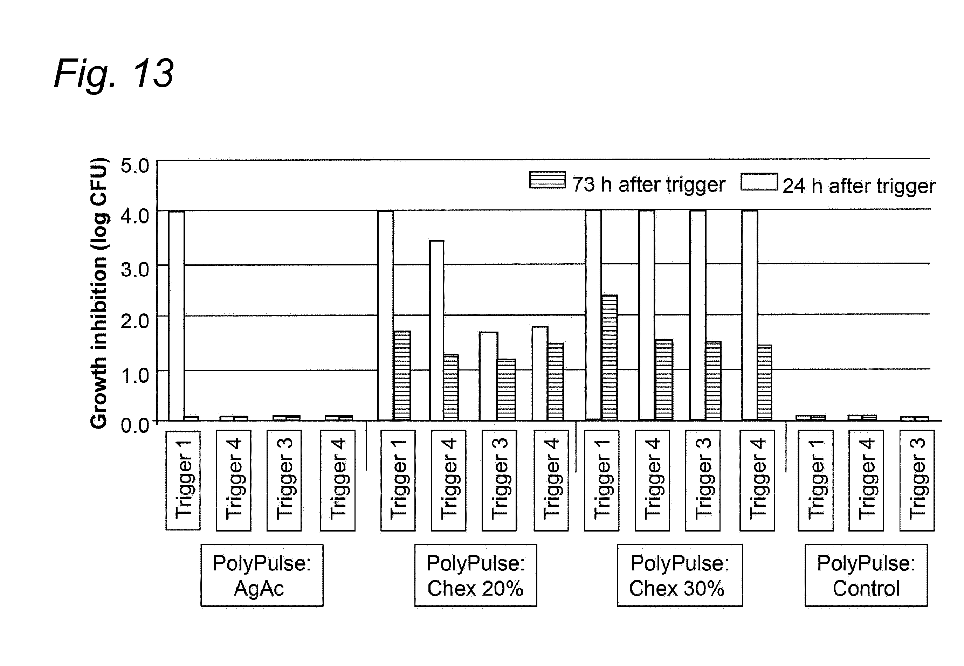

Figure 1 shows a release profile of silver ions from PS-BA 80/20 (PP1) as a function of time. Figure 2 shows a release profile of silver ions from PS-BA 80/20 (PP1) as a function of time (zoomed in from Figure 1). Figure 3 shows a release profile of mandelic acid from PS-BA 80/20 (PP1) as a function of time. Figure 4 shows a release profile of ibuprofen from PS-BA 78/22 (PP2) as a function of time. Figure 5 shows a release profile of chlorhexidine from PS-BA 71/29 (PP3) as a function of time, for PS-BA comprising 20 wt.% (lower curve) or 30 wt.% (upper curve) chlorhexidine. Figure 6 shows a release profile of chlorhexidine from PS-BA 80/20 (PP4) as a function of time, for PS-BA comprising 20 wt.% chlorhexidine. Figure 7 shows a release profile of ibuprofen from PS-BA 78/22 as a function of time, for PS-BA comprising 40 wt.% ibuprofen. Figure 8 shows the release rates of ibuprofen from PS-BA. Figure 9 shows a release profile of ibuprofen from PS-BMA 64/36 as a function of time, for PS-BA comprising 40 wt.% ibuprofen. Figure 10 shows release rates of ibuprofen from PS-BMA. Figure 11 shows release of Chlorhexidine from Tecothane TT 1095A (upper graph [●]), Tecophilic HP60D10 (middle graph[□]) and Tecoflex EG 93A (lower graph [■]). Figure 12 shows the procedure for antimicrobial activity assessment of antimicrobials released after a temperature trigger. Figure 13 shows growth inhibiting activity measured for samples with triggered release of Chlorhexidine (Chex) or Silver (AgAc) and for control samples, measured at 24h (light grey bars) and 73 h (dark grey bars) after each of four temperature triggers.

### Detailed description of the invention

### Definitions

The verb "to comprise" as is used in this description and in the claims and its conjugations is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

C₁-C₁₂ alkyl groups comprise 1 to 12 carbon atoms and have the general formula CₙH₂ₙ₊₁ and may be linear or branched. C₁-C₁₂ alkyl groups may also contain a cyclic moiety, and thus have the concomitant general formula CₙH₂ₙ₋₁. Examples of alkyl groups include methyl, ethyl, n-propyl, 2-propyl, n-butyl, t-butyl, n-hexyl, 2-hexyl, 3-hexyl, etc.

Within the framework of the present invention, the term "energy source" is to be understood to be a source of energy via (1) light waves, sound waves or microwaves, (2) electric current, (3) electrical, magnetic or radioactive radiation or (4) a combination thereof.

Also within the framework of the present invention, the term "active compound" is to be understood as a compound that provides, either directly or indirectly, a certain action. Preferably, the active compound provides a biological effect within a mammalian body. Hence, according to the invention, an active compound may be a medicament or pharmaceutical that has a direct biological effect on a mammalian body. But the invention is not regarded as limited to medicaments and pharmaceuticals so that the term "active compound" also includes substances like flavourings, odorants and colorants.

Further, within the framework of the invention, the glass transition temperature Tg is not limited to the glass transition temperature of the thermo-sensitive polymeric material *per* se. The glass transition temperature Tg may also denote the glass transition temperature of e.g. the combination of the thermo-sensitive polymeric material and the pharmaceutically or biologically active compound. In other words, the glass transition temperature as used herein does not refer to the glass transition temperature of the polymeric material *per se,* unless otherwise stated. In addition, the drug delivery device according to the present invention may comprise further components or additives that influence the glass transition temperature. Furthermore, when in use, for example as an implant, the drug delivery device according to the present invention may be in contact with further components, e.g. body fluids which are usually aqueous in nature, that effect the glass transition temperature of the drug delivery device. Consequently, the glass transition temperature Tg should be interpreted as the glass transition temperature of the thermo-sensitive part or region of the drug delivery device according to the present invention which comprises the thermo-sensitive polymeric material. As will become apparent below, the thermo-sensitive part or region of the drug delivery device according to the present invention may constitute a core, a sublayer or an outer layer of the drug delivery device. Preferably, the thermo-sensitive part or region is an outer layer, more preferably the outer layer, of the drug delivery device according to the invention. However, it will be apparent to the person skilled in the art that it is not necessary that the thermo-sensitive part or region is the outer layer, but that it may be surrounded by another layer as the outer layer, provided that this other -outer- layer is permeable for the pharmaceutically or biologically active component.

### Drug delivery device comprising a copolymer of styrene and C1-C12 alkyl (meth)acrylate

The present invention relates to a drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the characteristics of diffusion or transport of the active compound can be modified in a reversible manner by supplying energy from an energy source.

The present invention is based on the discovery that application of energy, e.g. light waves, sound waves, microwaves, electric current, electrical radiation, magnetic radiation, radioactive radiation, or a combination thereof, to a thermo-sensitive polymeric material comprising a copolymer of styrene and C₁-C₁₂ alkyl (meth)acrylate results in absorption of the energy through viscous shearing and relaxation which results in heating of the thermo-sensitive polymeric material. It was found that a thermo-sensitive polymeric material comprising a copolymer of styrene and C₁-C₁₂ alkyl (meth)acrylate provides very good pulsative drug release. In contrast, polystyrene homopolymers or poly(meth)acrylate homopolymers do not provide sufficient pulsatile drug release.

For example, if the energy is ultrasound, it appears that the temperature of the thermo-sensitive polymeric material increases linearly with the ultrasonic intensity. Moreover, the temperature increase is induced rapidly when the thermo-sensitive polymeric material is exposed to energy. This fast temperature increase is utilised to change the glassy state of the thermo-sensitive polymeric material into the rubbery state by heating the thermo-sensitive polymeric material from a temperature below the glass transition temperature T_{g} of the thermo-sensitive polymeric material to a temperature above the T_{g} of the thermo-sensitive polymeric material. In addition, at a temperature around the T_{g} the absorption of energy is substantial since mechanical damping has a maximum at this temperature. If the temperature of the thermo-sensitive polymeric material is below the Tg, release of the drug is extremely low whereas if the temperature of the thermo-sensitive polymeric material is above the Tg, flexible polymer chains allow for fast diffusion of the drug.

When the drug delivery device according to a preferred embodiment of the present invention is implanted in the body of a mammal, it is surrounded by an aqueous environment. In this aqueous environment, viscous shearing does not occur because energy is less absorbed by the water of the aqueous environment than by the thermo-sensitive polymeric material. Consequently, the drug delivery device according to the present invention does not have any harmful effect to a patient when it is exposed to energy.

If the energy employed is ultrasound, the ultimate temperature increase that occurs within the thermo-sensitive polymeric material is dependent from the ultrasound frequency and the attenuation coefficient of the thermo-sensitive polymeric material. Hence, according to the invention, the thermo-sensitive polymeric material has preferably an attenuation at 1 MHz of about 10 to about 1000 dB/cm, more preferably about 50 to about 500 dB/cm.

An advantage of the drug delivery device and method according to the present invention is that the transport characteristics can be reversibly modified in such a way that the diffusion of the active compound occurs repeatedly and reproducibly. In this way, the release of an active compound can be controlled as a function of time. Another advantage of the present invention is that an active compound can be delivered on demand.

The present invention therefore provides a drug delivery device of which the release characteristics of an active compound are modified on demand and/or are modified in a reversible manner by supplying energy from an energy source that optionally differs as a function of time. The release characteristics of an active compound by the drug delivery device are modified by supplying energy from an energy source to the drug delivery device. The energy source may for example be a source of light waves, sound waves, microwaves, electric current, electrical radiation, magnetic radiation, electromagnetic radiation, radioactive radiation, or a combination thereof. In a preferred embodiment, the energy source is a source of electric current, preferably a heating wire.

The thermo-sensitive part or region of the drug delivery device has in particular a T_{g} within a certain range, wherein supplying energy to the drug delivery device results in heating of the thermo-sensitive part or region above its T_{g} and in an enhanced release of the active component that is present.

The thermo-sensitive polymeric material (a) that is present in the drug delivery device according to the invention has a barrier effect against diffusion of an active compound and a glass transition temperature T_{g} within the range of about 0° to about 90°C. As was described above, the characteristics of diffusion or transport of the active compound (a) for the thermo-sensitive polymeric material (b) can be modified in a reversible manner. An advantage of the drug delivery device and method according to the present invention is that the release characteristics can be reversibly modified in such a way that the release of the active component occurs repeatedly and reproducibly. In this way, the release of an active component can be controlled as a function of time. Another advantage of the present invention is that an active component can be delivered on demand.

According to the invention, it is preferred that the thermo-sensitive polymeric part or region of the drug delivery device has a T_{g} in the range of about 0 to about 90°C, more preferably in the range of about 30 to about 90°C, even more preferably in the range of about 35 to about 85°C, even more preferably in the range of about 40 to about 80°C, and most preferably in the range of about 45 to about 75°C.

The thermo-sensitive polymeric material that is present in the drug delivery device according to the invention comprises a copolymer of styrene and C₁-C₁₂ alkyl (meth)acrylate, herein also referred to as poly(styrene/ C₁-C₁₂ alkyl (meth)acrylate). The term C₁-C₁₂ alkyl (meth)acrylate is meant to include C₁-C₁₂ alkyl acrylate and C₁-C₁₂ alkyl methacrylate. A copolymer of styrene and C₁-C₁₂ alkyl (meth)acrylate herein refers to a copolymer of styrene and C₁-C₁₂ alkyl acrylate, a copolymer of styrene and C₁-C₁₂ alkyl methacrylate, a copolymer of styrene, C₁-C₁₂ alkyl methacrylate and C₁-C₁₂ alkyl acrylate, or mixtures thereof. A copolymer of styrene and C₁-C₁₂ alkyl (meth)acrylate is herein defined as a copolymer that is obtained by copolymerization of styrene monomer and C₁-C₁₂ alkyl (meth)acrylate monomer. In one embodiment, said copolymer of styrene and C₁-C₁₂ alkyl (meth)acrylate is a statistical copolymer. In another embodiment said copolymer is a block copolymer, preferably a diblock or a triblock copolymer. Alternating, random and block copolymers of styrene and C₁-C₁₂ alkyl (meth)acrylate are known in the art, as is the way of preparation of said copolymers. The copolymer of styrene and C₁-C₁₂ alkyl (meth)acrylate may for example be prepared via a free radical polymerization process, e.g. a solution polymerization process, a suspension polymerization process or an emulsion polymerization process. Preferably, said copolymer is prepared via a solution polymerization process.

Preferably, said copolymer of styrene and C₁-C₁₂ alkyl (meth)acrylate is a copolymer of styrene and C₁-C₁₂ alkyl acrylate or a copolymer of styrene and C₁-C₁₂ alkyl methacrylate, more preferably a copolymer of styrene and C₁-C₁₂ alkyl acrylate. More preferably, said copolymer is a random copolymer.

Preferably, the C₁-C₁₂ alkyl (meth)acrylate is a C₁-C₆ alkyl (meth)acrylate. The alkyl group is preferably a methyl, ethyl, propyl, butyl, pentyl or hexyl group, more preferably a methyl, ethyl, n-propyl, n-butyl, n-pentyl or n-hexyl group. Most preferably, the alkyl group is an n-butyl group. Said copolymer of styrene and C₁-C₁₂ alkyl (meth)acrylate is thus preferably a copolymer of styrene and n-butyl (meth)acrylate, and more preferably a copolymer of styrene and n-butyl acrylate.

In a preferred embodiment, said copolymer of styrene and C₁-C₁₂ alkyl (meth)acrylate has a number average molecular weight Mₙ in the range of about 3,000 to about 500,000 g/mol, preferably in the range of about 5000 to about 250,000 g/mol, more preferably in the range of about 8,000 to about 150,000 g/mol and most preferably in the range of about 10,000 to about 100,000 g/mol.

In another preferred embodiment, said copolymer has a polydispersity index PDI in the range of about 0.5 to about 4, preferably in the range of about 0.6 to about 3, more preferably in the range of about 0.7 to about 2.5 and most prefererably in the range of about 0.8 to about 2.0.

In a preferred embodiment, the ratio of styrene/ C₁-C₁₂ alkyl (meth)acrylate in said copolymer of styrene and C₁-C₁₂ alkyl (meth)acrylate is in the range of about 5/95 to about 95/5 mol% styrene/ C₁-C₁₂ alkyl (meth)acrylate, preferably in the range of about 95/5 to about 50/50 mol%, more preferably in the range of about 95/5 to about 60/40 mol%, more preferably in the range of about 90/10 to about 70/30 mol% and most preferably in the range of about 85/15 to about 75/25 mol% styrene/ C₁-C₁₂ alkyl (meth)acrylate.Said copolymer preferably has a glass transition temperature T_{g} in the range of about 0 to about 90°C, more preferably in the range of about 30 to about 90°C, even more preferably in the range of about 35 to about 85°C, even more preferably in the range of about 40 to about 80°C, and most preferably in the range of about 45 to about 75°C.

The thermo-sensitive polymeric material (b) that is present in the drug delivery device according to the invention comprises a copolymer of styrene and C₁-C₁₂ alkyl (meth)acrylate, preferably of styrene and C₁-C₆ alkyl (meth)acrylate, more preferably of styrene and n-butyl (meth)acrylate, and most preferably a copolymer of styrene and n-butyl acrylate. Preferably, said thermo-sensitive polymeric material comprises about 10 wt.% or more, more preferably about 30 wt.% or more, even pore preferably about 50 wt.% or more, even more preferably about 70 wt.% or more, and even more preferably about 80 wt.% or more of said copolymer of styrene and C₁-C₁₂ alkyl (meth)acrylate, preferably of styrene and C₁-C₆ alkyl (meth)acrylate, more preferably of styrene and n-butyl (meth)acrylate, and most preferably of styrene and n-butyl acrylate, based on the total weight of the thermo-sensitive polymeric material. Yet even more preferably, the thermo-sensitive polymeric material comprises about 90 wt.% or more, even more preferably about 95 wt.% or more of the copolymer of styrene and C₁-C₁₂ alkyl (meth)acrylate, preferably of styrene and C₁-C₆ alkyl (meth)acrylate, more preferably of styrene and n-butyl (meth)acrylate, and most preferably a copolymer of styrene and n-butyl acrylate, based on the total weight of the thermo-sensitive polymeric material. Most preferably, said thermo-sensitive polymeric material is a copolymer of styrene and C₁-C₁₂ alkyl (meth)acrylate, preferably of styrene and C₁-C₆ alkyl (meth)acrylate, more preferably of styrene and n-butyl (meth)acrylate, and most preferably a copolymer of styrene and n-butyl acrylate. Preferred embodiments of the copolymer of styrene and C₁-C₁₂ alkyl acrylate are described in detail above.

The use of a thermo-sensitive material comprising a copolymer of styrene and C₁-C₁₂ alkyl (meth)acrylate in a drug delivery device according to the invention has several advantages. As was mentioned earlier, an advantage of the drug delivery device and the method for releasing an active compound from a drug delivery device according to the invention is that the thermo-sensitive material comprising a copolymer of styrene and C₁-C₁₂ alkyl (meth)acrylate has advantageous characteristics as compared to thermo-sensitive polymers used in drug delivery devices known in the art. Said thermo-sensitive material comprising a copolymer of styrene and C₁-C₁₂ alkyl (meth)acrylate has for example an enhanced diffusion of active compound when heated above its glass transition temperature Tg, as well as an improved barrier effect against diffusion of an active compound at temperatures below its Tg.

For example, when the thermo-sensitive polymeric material is a copolymer of polystyrene and C₁-C₁₂ alkyl acrylate, said thermo-sensitive polymeric material has an improved barrier effect against diffusion of an active compound at a temperature below its glass transition temperature Tg, e.g. at 37°C (i.e. human body temperature, a relevant temperature for a drug delivery device implanted in a mammal), ensuring that release of the active compound does not occur at normal body temperature. At a temperature above its T_{g} said thermo-sensitive polymeric material still releases a sufficient amount of active compound. (when used in the right proportions).

The properties of the thermo-sensitive polymeric material may be adjusted by varying the proportions of monomers in the copolymer comprising styrene and C₁-C₁₂ alkyl (meth)acrylate. For example, when the proportion of styrene is increased, a more rigid copolymer is obtained, whereas a more flexible copolymer is obtained by increasing the proportion of C₁-C₁₂ alkyl acrylate and/or C₁-C₁₂ alkyl acrylate. By varying the composition of these two monomers in the copolymer, the properties of the thermo-sensitive polymeric material can be adjusted. When the material becomes too soft or too brittle, e.g. when loaded with a certain amount of active compound, these properties can be adjusted by varying the proportion of monomers within the copolymer.

Another advantage of the use of a thermo-sensitive polymeric material comprising a copolymer of styrene and C₁-C₁₂ alkyl acrylate is that the active compounds generally have a high maximum solubility in this copolymer.

Depending on the therapeutic or diagnostic applications and the active compound concerned, the drug delivery device according to the invention can be made in various ways.

According to a first preferred embodiment of the drug delivery device according the invention the active compound (a) is mixed, preferably in a homogeneous manner, with the thermo-sensitive polymeric material (b). In this first preferred embodiment, the active compound (a) mixed with the thermo-sensitive polymeric material (b) together form the thermo-sensitive part or region of the drug delivery device. In a further preferred embodiment, said active compound (a) is homogeneously dispersed in the thermo-sensitive polymeric material (b).

In a second preferred embodiment, the drug delivery device according to the invention comprises particles comprising the active compound (a) and an inert support material, wherein said particles are coated with a layer of said thermo-sensitive polymeric material (b). In this second preferred embodiment, the coating of said particles forms the thermo-sensitive part or region of the drug delivery device. Although said particles may have any shape, preferably the particles have a spherical shape. The preferred size of said particles is in the range of about 10 µm to about 1,5 mm, more preferably in the range of about 30 to about 600 µm. The inert support material may for example comprise polycaprolactone (PCL), polycaprolactonediol (PCL-iol), or a mixture thereof.

In a third preferred embodiment of the drug delivery device according to the invention, the active compound (a) is comprised by the core of the drug delivery device and the core is surrounded by a layer comprising the thermo-sensitive polymeric material (b). In this third preferred embodiment, the layer comprising the thermo-sensitive polymeric material forms the thermo-sensitive part of the drug delivery device.

In particular when the drug delivery device according to the invention is implanted in a mammalian body, it is preferred that the drug delivery device is surrounded by an enveloping material that is thermally insulating and that is permeable for the active compound, in order to protect the tissues and/or the organs in which the drug delivery device is present from the energy supplied. Therefore, the drug delivery device according to the invention optionally further comprises a material (c) being thermally insulating and permeable for the active compound.

The present invention thus also relates to a drug delivery device, in particular for implantation in a mammalian body, comprising:
(a) an active compound,
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C, and
(c) a material being thermally insulating and permeable for the active compound,
wherein the characteristics of diffusion or transport of the active compound can be modified in a reversible manner by supplying energy from an energy source.

Preferred embodiments of active compound (a) are described in more detail below, and preferred embodiments of thermo-sensitive polymeric material (b) are described in more detail above. Also in this embodiment, it is preferred that the thermo-sensitive polymeric material (b) that is present in the drug delivery device according to the invention comprises a copolymer of styrene and and C₁-C₆ alkyl (meth)acrylate, more preferably of styrene and n-butyl (meth)acrylate, and most preferably a copolymer of styrene and n-butyl acrylate.

In a preferred embodiment, the material (c) being thermally insulating and permeable for the active compound envelopes, i.e. surrounds, active compound (a) and thermo-sensitive polymeric material (b).

In a preferred embodiment, the material (c) being thermally insulating and permeable for the active compound is a hydrogel. Hydrogels are three dimensional networks of hydrophilic polymers in which a large amount of water is present. In general the amount of water present in a hydrogel is at least 20 weight percent of the total weight of the dry polymer. The most characteristic property of these hydrogels is that they swell in the presence of water and shrink in the absence of water. The extent of swelling (equilibrium water content) is determined by the nature (mainly the hydrophilicity) of the polymer chains and the crosslinking density. Polymers that can be used for manufacturing hydrogels are well known in the art and are for example disclosed in US 2.340.110, US 2.340.111, US 2.533.635, US 2.798.053, US 3.940.351, US 4.062.817, US 5.034.486, US 5.034.487, US 5.034.488 and US 5.468.797, all incorporated by reference herein. Another type of hydrogel is based on supramolecular host-guest chemistry, e.g. a hydrogel made by reversible cross-linking of an acryl amide polymer having pendant adamantyl groups with a cyclodextrine dimer as disclosed by Kretschmann *et al.,* Angew. Chem. Int. Ed. Engl. *45*, 4361 - 4365, **2006.**

In another preferred embodiment, said material (c) being thermally insulating and permeable for the active compound is a thermoplastic polyurethane (TPU), preferably an aromatic TPU, and more preferably an aromatic polyether-based TPU.

Thermoplastic polyurethanes are known in the art. TPUs are formed by the reaction of a diisocyanate, a short-chain diol (also called chain extender) and long-chain bifunctional diols (known as polyols). A TPU is composed of hard and soft segments, wherein the hard segment may be aromatic or aliphatic. Aromatic TPUs may be based on aromatic isocyanates. Aromatic isocyanates are well-known in the art, and include for example methylene diphenyl diisocyanate (MDI), toluene diisocyanate (TDI), meta- or para-phenylene diisocyanate and 1,5-naphtalenediisocyanate. Aliphatic TPUs may be based on aliphatic isocyanates, which are well-known in the art and include for example hexamethylene diisocyanate (HDI), dicyclohexylmethane 4,4'-diisocyanate (H12MDI) and isophorone diisocyanate (IPDI). The hard segment of a TPU is composed of the combination of a diisocyanate with a short-chain diol. The soft sement of a TPU is composed of the polyol, and may be polyester-based or polyether-based. The soft segment may also be based on a combination of polyester and polyether polyols. Examples of polyesters include adipates, polycaprolactones, aliphatic polycarbonates. Examples of polyethers include poly(oxypropylene)diols, poly(oxytetramethylene)diols. Aromatic polyether-based TPUs are well known in the art, and for example disclosed in WO 2004/078816, WO 2004/078816 and WO 2008/089163, all incorporated by reference herein.

Examples of aliphatic TPUs include Tecoflex® TPUs, for example Tecoflex® EG 93A, and Tecophilic®, all commercially available from Lubrizol.

Preferably, the aromatic polyether-based thermoplastic polyurethane is Tecothane®, more preferably Tecothane® TT-1095A, commercially available from Lubrizol. Use of Tecothane® TT-1095A as material (c) being thermally insulating and permeable for the active compound has several advantages. Tecothane® TT-1095A has a very good biocompatibility, and good thermal insulation properties. In addition, Tecothane® TT-1095A has a very good permeability for the active compound. Use of Tecothane® TT-1095A is particularly advantageous when said active compound comprises chlorhexidine or a source of chlorhexidine ions.

The present invention therefore further relates to a drug delivery device, comprising:
(i) a central region comprising said active compound (a) and said thermo-sensitive polymeric material (b), and
(ii) an enveloping material (c) surrounding said central region, said enveloping material being thermally insulating and permeable for said active compound.

Preferred embodiments of active compound (a) are described in more detail below, and preferred embodiments of thermo-sensitive polymeric material (b) are described in more detail above. Also in this embodiment, it is preferred that the thermo-sensitive polymeric material (b) that is present in the drug delivery device according to the invention comprises a copolymer of styrene and and C₁-C₆ alkyl (meth)acrylate, more preferably of styrene and n-butyl (meth)acrylate, and most preferably a copolymer of styrene and n-butyl acrylate.

The drug delivery device according to the invention may comprise more than one central region (i) and thus more than one enveloping material (ii) surrounding said central region.

Said central region comprising the active compound (a) and the thermo-sensitive polymeric material (b) may for example be according to the preferred embodiments as described above. Therefore, in a preferred embodiment, the drug delivery device according to the invention comprises a central region (i) wherein the active compound (a) is mixed, preferably in a homogeneous manner, with the thermo-sensitive polymeric material (b). In another preferred embodiment, the drug delivery device according to the invention comprises a central region (i), wherein said central region comprises particles comprising the active compound (a) and an inert support material, wherein said particles are coated with a layer of said thermo-sensitive polymeric material (b). In yet another preferred embodiment, the drug delivery device according to the invention comprises a central region (i) wherein the active compound (a), and optionally an inert support material, is comprised by the core of the drug delivery device and said core is surrounded by a layer comprising the thermo-sensitive polymeric material (b).

An advantage of the presence of an enveloping material (c) surrounding the central region is that the environment in which the drug delivery device is present, in particular the physiological environment of the mammalian body, can be protected from changes in the drug delivery device that are a consequence of the supply of energy. One example of this is protection from a rise in the temperature of the drug delivery device. For example, when the drug delivery device according to the invention is implanted in a mammalian body, the presence of a thermally insulating enveloping material protects the surrounding tissue from the temperature increase of the thermo-sensitive part of the drug delivery device caused by the energy supply to the drug delivery device needed to release the active compound.

In addition, the use of an enveloping material being thermally insulating and permeable for the active compound has as further advantages that it can counteract fouling of the thermo-sensitive polymeric material. It can further provide more strength to the drug delivery device and provide flexibility thereto and inhibits negative influences from sarcophagus and aggressive bacteria. It further provides the option that thermo-sensitive polymeric materials can be used which are less biocompatible (rejections) provided that the enveloping material is sufficiently biocompatible.

The enveloping material (c) surrounding the central region may be selected from a wide range of materials and structures, including for example the wall of a catheter (wherein the drug delivery device is e.g. incorporated in the wall of the catheter). In a preferred embodiment, said enveloping material (c) comprises a hydrogel. Hydrogels are described in more detail above. In another preferred embodiment, said enveloping material (c) comprises an aromatic polyether-based thermoplastic polyurethane (TPU), preferably Tecothane®, more preferably Tecothane® TT-1095A, as described in more detail above. Advantages of the use of Tecothane® TT-1095A as enveloping material (c) being thermally insulating and permeable for the active compound are described in more detail above.

In yet another preferred embodiment, said enveloping material (c) is the wall of a catheter. In this embodiment, it is preferred that the enveloping material (c) is selected from the group of materials known in the art as being suitable for use in the wall of a catheter, provided that said material is sufficiently thermally insulating and permeable for the active compound (a).

The precise shape of the drug delivery device according to the invention, and therefore the precise shape (e.g. a sphere, a slab, a film, etc.) of the central region (i) and the enveloping material (c) surrounding the central region are governed by the requirements of the particular drug delivery device.

Particularly advantageous applications of the present invention are in the field of the release of pharmaceutically or biologically active compounds. An active compound herein refers to a pharmacological and/or biological substance, i.e. a substance that is biologically and/or pharmaceutically active, for example a drug or a prodrug. The active compound (a) that is present in the drug delivery device according to the invention may for example be selected from the group consisting of drugs and prodrugs, diagnostic agents and contrast media for imaging. Preferably, the active compound (a) is a drug or a prodrug, more preferably a drug or a prodrug selected from the group consisting of chemotherapeutic agents, analgesics, anaesthetics, hormonal substances, antimicrobial agents, infection suppressing agents and antiarrhythmic agents. In a further preferred embodiment, the active compound is selected from the group of antimicrobial agents. More preferably the active compound comprises chlorhexidine or a source of chlorhexidine ions, mandelic acid and/or a source of silver ions Ag⁺. Sources of chlorhexidine ions are known in the art, and include for example chlorhexidine diacetate, chlorhexidine digluconate, chlorhexidine hydrochloride and the like. Sources of silver ions are known in the art, and include for example silver acetate, silver nitrate, silver chloride and silver carbonate. The active compound preferably comprises chlorhexidine or a source of chlorhexidine ions. In a further preferred embodiment, said active compound (a) comprises a mixture of chlorhexidine or a source of chlorhexidine ions, and silver acetate.

In an embodiment wherein the active compound (a) is mixed with the thermo-sensitive polymeric material (b), as described above, it is preferred that the thermo-sensitive polymeric material comprises about 5 to about 50 wt.%, preferably about 10 to about 45 wt.%, more preferably about 15 to about 40 wt.% and most preferably about 15 to about 35 wt.% of the active compound (a), based on the total weight of (a) plus (b). In an embodiment wherein the active compound (a) is comprised by the core of the drug delivery device, wherein said core is surrounded by a layer of said thermo-sensitive polymeric material, as described above, the amount of active compound (a) present in the thermo-sensitive polymeric material (b) is determined by the solubility of the active compound in the thermo-sensitive polymer.

The drug delivery device according to the present invention may for example be used to control post-operative heart rhythm complaints wherein the drug delivery device including the appropriate medicament is implanted in the pericardium.

The present invention also relates to a carrier comprising a drug delivery device according to the invention. The carrier may for example be a catheter, a dermal patch or a porous web-like material, woven or non-woven. In a preferred embodiment, said carrier is a catheter. The invention therefore also relates to a catheter, comprising a drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the characteristics of diffusion or transport of the active compound can be modified in a reversible manner by supplying energy from an energy source. In a further preferred embodiment, said catheter further comprises an energy source, preferably a heating means, for example a heating filament.

Also in this embodiment, it is preferred that the thermo-sensitive polymeric material (b) that is present in the drug delivery device according to the invention comprises a copolymer of styrene and and C₁-C₆ alkyl (meth)acrylate, more preferably of styrene and n-butyl (meth)acrylate, and most preferably a copolymer of styrene and n-butyl acrylate.

In a preferred embodiment, the invention relates to a catheter comprising a drug delivery device according to the invention, comprising:
(i) a central region comprising said active compound (a) and said thermo-sensitive polymeric material (b), and
(ii) an enveloping material (c) surrounding said central region, said enveloping material being thermally insulating and permeable for the active compound.

Preferred embodiments of the drug delivery device according to the invention, including preferred embodiments of the thermo-sensitive polymeric material comprising a thermo-sensitive polymeric material comprising a copolymer of styrene and C₁-C₁₂ alkyl (meth)acrylate and preferred embodiments thereof, are described in detail above. As in the drug delivery device according to the invention, in the catheter according to the invention it is preferred that the thermo-sensitive polymeric material (b) comprises a copolymer of styrene and C₁-C₆ alkyl (meth)acrylate, more preferably of styrene and n-butyl (meth)acrylate, and most preferably a copolymer of styrene and n-butyl acrylate.

In a further preferred embodiment, the catheter according to the invention further comprises an energy source, preferably a heating means, for example a heating filament.

In these embodiments, the enveloping material (c) may for example be the wall of the catheter, or be comprised in the wall of the catheter. The drug delivery device according to the invention may for example be incorporated in the wall of said catheter.

In these embodiments, it is preferred that the enveloping material (c) is selected from the group of materials known in the art as being suitable for use as the wall of a catheter, provided that said material is sufficiently thermally insulating, and permeable for the active compound (a).

In a further preferred embodiment, it is preferred that said enveloping material being thermally insulating and permeable for said active compound comprises an aromatic polyether-based thermoplastic polyurethane (TPU), as described in more detail above. In a further preferred embodiment, said TPU comprises Tecothane®, more preferably Tecothane® TT-1095A, as described in more detail above. More preferably, said enveloping material being thermally insulating and permeable for said active compound is Tecothane®, even more preferably Tecothane® TT-1095A.

As in the drug delivery device according to the invention, in the catheter according to the invention it is also preferred that the active compound is selected from the group consisting of chemotherapeutic agents, analgesics, anaesthetics, hormonal substances, antimicrobial agents, infection suppressing agents and antiarrhythmic agents. In a further preferred embodiment the active compound is selected from the group of antimicrobial agents. More preferably the active compound comprises chlorhexidine, mandelic acid and/or a source of silver ions Ag⁺. Sources of silver ions are known in the art, and include for example silver acetate, silver nitrate, silver chloride and silver carbonate. The active compound preferably comprises chlorhexidine or a source of chlorhexidine ions. In a further preferred embodiment, said active compound (a) comprises chlorhexidine or a source of chlorhexidine ions, and a source of silver ions, preferably silver acetate.

The invention relates in particular to a catheter, comprising a heating means and a drug delivery device according to the invention, said drug delivery device comprising:
(i) a central region comprising said active compound (a) and said thermo-sensitive polymeric material (b), and
(ii) an enveloping material (c) surrounding said central region, said enveloping material being thermally insulating and permeable for the active compound,
wherein the characteristics of diffusion or transport of the active compound can be modified in a reversible manner by supplying energy from said heating means, and wherein the active compound (a) is selected from the group consisting of chlorhexidine or a source of chlorhexidine ions, mandelic acid and a source of silver ions.

The invention thus also relates to a catheter, provided with a layer of a thermo-sensitive polymeric material (b), wherein said thermo-sensitive polymeric material comprises an active compound (a), and wherein the carrier is also provided with a heating means, for example a heating filament. Such catheters are for example disclosed in US 6,740,108, incorporated by reference herein. US 6,740,108 discloses a thermal therapy catheter for preferentially treating tissue adjacent to a body lumen. In US 6,740,108 a catheter is discosed, the catheter comprising (A) a catheter shaft having an outer surface, the catheter shaft being insertable into a body lumen, (B) an energy-emitting element carried by the catheter shaft, the energy-emitting element being operable to radiate a generally symmetrical energy pattern, (C) a plurality of cooling lumens in the catheter shaft around the energy-emitting element, the plurality of cooling lumens being configured for circulation of a fluid therethrough, and (D) means located in at least one of the plurality of cooling lumens for attenuating energy radiated by the energy-emitting element.

According to yet another embodiment of the invention, particles comprising the thermo-sensitive polymeric material and the active compound can occur in a form that can be administered by injection, e.g. intravenously or intramuscularly, as is for example disclosed in US 2003/0212148, incorporated by reference herein.

The present invention further relates to the use of a drug delivery device according to the invention for implantation in a mammalian body, to the use of a drug delivery device according to the invention for incorporation into a prosthesis or an artificial organ, and to the use of the drug delivery device according to the invention for incorporation into a catheter.

The present invention further relates to the use of an aromatic polyether-based thermoplastic polyurethane (TPU), preferably Tecothane®, more preferably Tecothane® TT-1095A, as a thermally insulating material in a drug delivery device, preferably a drug delivery device according to the present invention. Said drug delivery device according to the present invention is described in more detail above.

### Drug delivery device comprising a TPU as thermally insulating material

The invention further relates to a drug delivery device comprising:
(a) an active compound,
(b) a thermo-sensitive polymeric material having a barrier effect against diffusion of an active compound and having a glass transition temperature within the range of about 0° to about 90°C, and
(c) a material being thermally insulating and permeable for the active compound, wherein said material being thermally insulating and permeable for the active compound comprises an aromatic polyether-based thermoplastic polyurethane (TPU),
wherein the characteristics of diffusion or transport of the active compound can be modified in a reversible manner by supplying energy from an energy source.

Active compound (a) and thermo-sensitive material (b) are described in more detail below).

Said material (c) being thermally insulating and permeable for the active compound comprises a thermoplastic polyurethane (TPU), preferably an aromatic TPU, and more preferably an aromatic polyether-based TPU.

Thermoplastic polyurethanes are known in the art. TPUs are formed by the reaction of a diisocyanate, a short-chain diol (also called chain extender) and long-chain bifunctional diols (known as polyols). A TPU is composed of hard and soft segments, wherein the hard segment may be aromatic or aliphatic. Aromatic TPUs may be based on aromatic isocyanates. Aromatic isocyanates are well-known in the art, and include for example methylene diphenyl diisocyanate (MDI), toluene diisocyanate (TDI), meta- or para-phenylene diisocyanate and 1,5-naphtalenediisocyanate. Aliphatic TPUs may be based on aliphatic isocyanates, which are well-known in the art and include for example hexamethylene diisocyanate (HDI), dicyclohexylmethane 4,4'-diisocyanate (H12MDI) and isophorone diisocyanate (IPDI). The hard segment of a TPU is composed of the combination of a diisocyanate with a short-chain diol. The soft sement of a TPU is composed of the polyol, and may be polyester-based or polyether-based. The soft segment may also be based on a combination of polyester and polyether polyols. Examples of polyesters include adipates, polycaprolactones, aliphatic polycarbonates. Examples of polyethers include poly(oxypropylene)diols, poly(oxytetramethylene)diols. Aromatic polyether-based TPUs are well known in the art, and for example disclosed in WO 2004/078816, WO 2004/078816 and WO 2008/089163, all incorporated by reference herein.

Examples of aliphatic TPUs include Tecoflex® TPUs, for example Tecoflex® EG 93A, and Tecophilic®, all commercially available from Lubrizol.
Preferably, the aromatic polyether-based thermoplastic polyurethane is Tecothane®, more preferably Tecothane® TT-1095A, commercially available from Lubrizol. Use of Tecothane® TT-1095A as material (c) being thermally insulating and permeable for the active compound has several advantages. Tecothane® TT-1095A has a very good biocompatibility, and good thermal insulation properties. In addition, Tecothane® TT-1095A has a very good permeability for the active compound. Use of Tecothane® TT-1095A is particularly advantageous when said active compound comprises chlorhexidine or a source of chlorhexidine ions.

Said aromatic polyether-based thermoplastic polyurethane (TPU) is preferably Tecothane®, more preferably Tecothane® TT-1095A, commercially available from Lubrizol.

The invention thus also relates to a drug delivery device comprising:
(i) a core comprising said active compound (a) and said thermo-sensitive polymeric material (b), and
(ii) an enveloping material (c) being thermally insulating and permeable for the active compound,
wherein said enveloping material comprises an aromatic polyether-based thermoplastic polyurethane (TPU).

The active compound (a) that is present in the drug delivery device according to this particular embodiment may for example be selected from the group consisting of drugs and prodrugs, diagnostic agents and contrast media for imaging. Preferably, the active compound (a) is a drug or a prodrug, more preferably a drug or a prodrug selected from the group consisting of chemotherapeutic agents, analgesics, anaesthetics, hormonal substances, antimicrobial agents, infection suppressing agents and antiarrhythmic agents. In a further preferred embodiment, the active compound is selected from the group of antimicrobial agents. More preferably the active compound comprises chlorhexidine or a source of chlorhexidine ions, mandelic acid and/or a source of silver ions Ag⁺. Sources of chlorhexidine ions are known in the art, and include for example chlorhexidine diacetate, chlorhexidine digluconate, chlorhexidine hydrochloride and the like. Sources of silver ions are known in the art, and include for example silver acetate, silver nitrate, silver chloride and silver carbonate. The active compound preferably comprises chlorhexidine or a source of chlorhexidine ions. In a further preferred embodiment, said active compound (a) comprises a mixture of chlorhexidine or a source of chlorhexidine ions, and silver acetate.

In this particular embodiment of the drug delivery device according to the invention it is preferred that a poly(lactic acid-co-glycolic acid), polymethyl methacrylate, poly(ethylene-co-vinyl acetate) or a nylon is used as the thermo-sensitive polymeric material (b). However, in this particular embodiment it is even more preferred that the thermo-sensitive polymeric material comprises a polymer or a copolymer comprising a monomer selected from the group consisting of a hydroxyl alkanoate wherein the alkyl group comprises 1 to 12 carbon atoms, lactide, glycolide, ε-caprolactone, 1,4-dioxane-2-one, 1,5-dioxepan-2-one, trimethylene carbonate (1,3-dioxane-2-one) and mixtures thereof. Preferably, for this particular embodiment, the copolymer is a random copolymer or a block copolymer and the block copolymer is preferably a diblock copolymer or a triblock copolymer. Such polymers and copolymers are well known in the art and are for example disclosed in US 2.668.162, US 2.703.316, US 3.636.956, US 3.839,297, US 4.137.921, US 4.157.437, US 4.243.775, US 4.443.430, US 5.076.983, US 5.310.865 and US 6.025.458, all incorporated by reference herein. This group of even more preferred thermo-sensitive polymeric materials for this particular embodiment also includes (meth)acrylic (co)polymers, polyester urethanes, polyester amides, polyether esters such as polyethylene glycol terephtalate - polybutylene terephalate (PEGT/PBT), polyethylene glycol and the natural polymers such as polyhydralonic acid, iso-sorbide, dextran, collagens and mixtures thereof. Another even more preferred thermo-sensitive polymeric material for this particular embodiment is also polybutyl methacrylate.

### Method for the release of an active compound

The present invention also relates to a method for releasing an active compound from a drug delivery device according to the invention, the drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the active compound is released from the drug delivery device by reversibly modifying the transport characteristics of the thermo-sensitive polymeric material by supplying energy from an energy source.

Preferably, the drug delivery device is for implantation in a mammalian body. An important advantage of the method according to the invention is that a pharmaceutically or biologically active component can be repeatedly released from the drug delivery device in a controlled manner as a function of time, depending on the therapeutic or diagnostic needs (reversible release). It is preferred that a medicament selected from the group of chemotherapeutic agents, analgesics, anesthetics, hormonal substances, infection suppressing agents, anti-microbial agents and anti-arrhythmic agents is used as active component. It is likewise preferred that a diagnostic agent or a contrast medium for imaging is used as active component.

Also in the method for releasing an active compound according to the invention, it is preferred that the thermo-sensitive polymeric material (b) that is present in the drug delivery device according to the invention comprises a copolymer of styrene and and C₁₂-C₆ alkyl (meth)acrylate, preferably a copolymer of styrene and C₁-C₆ alkyl (meth)acrylate, more preferably of styrene and n-butyl (meth)acrylate, and most preferably a copolymer of styrene and n-butyl acrylate.

In a preferred embodiment of the method according to the invention, the active compound is selected from the group consisting of chlorhexidine or a source of chlorhexidine ions, mandelic acid and a source of silver ions, as described in more detail above. In a further preferred method the active compound comprises chlorhexidine or a source of chlorhexidine ions, preferably in combination with a source of silver ions Ag⁺, preferably silver acetate.

Additional preferred embodiments of the drug delivery device according to the invention are described in detail above.

According to a preferred embodiment of the present invention, the energy is supplied from an energy source that is external with respect to the drug delivery device. According to this invention, the transport characteristics of the polymer can be modified remotely.

According to the invention, the energy may origin from many sources as explained above. The energy source may for example a source of energy via (1) light waves, sound waves or microwaves, (2) electric current, (3) electrical, magnetic or radioactive radiation or (4) a combination thereof.

In a preferred embodiment, the energy source is a source of electric energy, preferably electric current. In a further preferred embodiment the heating source comprises a heating wire or a heating filament.

If the energy to be employed is magnetism, the thermo-sensitive polymeric material must contain a magnetic material, for example thermo-sensitive magnetic particles as are disclosed in Chan et al., "Scientific and Clinical Applications of Magnetic Carriers", Häfeli et al. (Eds.), pages 607 - 617 Plenum Press, 1997, incorporated by reference herein. Alternatively, nanotubes may be included to render the thermo-sensitive polymeric material conductive so that when a magnetic field is applied an induced current is generated. As a result of the resistance that is encountered by this induced current, the thermo-sensitive polymeric material is heated (cf. Iijima, S., Helical microtubes of graphitic carbon, Nature, Vol. 354, 56 - 58; Iijima, S., Ichihasi, T., Single-shell carbon nanotubes of 1-nm diameter, Nature, Vol 363, 1993, 603 - 605; Lambin, P., Electronic structure of carbon nanotubes, C.R. Physique, Vol. 4, 2003, 1009 - 1019; Dresselhaus, M., Dresselhaus, G., Eklund, P., Saito, R., Carbon nanotubes, Physics World, Vol. 11, Issue 1, 1998, 33 - 38; Kiang, C., Goddard, W.A., Beyers, R., Bethune, D.S., Carbon nanotubes with single-layer walls, Carbon, Vol 33, Issue 7, 903 - 914; all incorporated by reference herein). The ultimate temperature increase that occurs within the magnetic material is measured in the Specific Power Absorption Rate (SAR) - cf. Chan *et al. -* wherein the optimum ranges are within the range of 500 - 600 W/g. According to the invention, it is preferred that about 3 to about 30 mg, preferably about 5 to about 15 mg, thermo-sensitive magnetic particles are incorporated in the thermo-sensitive polymeric material per g thermo-sensitive polymeric material.

If the energy to be employed is infrared light, the ultimate temperature increase that occurs within the thermo-sensitive polymeric material is dependent on the intensity and wavelength of the infra red light as will be understood by the person skilled in the art. Obviously, a specific inter-atomic bond of the polymer can for example be activated by using a specific wave length. In this embodiment, it is preferred that the drug delivery device according to the invention further comprises an infrared absorbing compound. Infrared absorbing compounds are known in the art, and include for example so-called "rylenes" (perylenes, terrylenes, quaterrylenes, etc.) and carbon compounds, provided that the infrared absorbing compound is capable of absorbing infrared irradiation having a wave length in the range of about 500 nm to 1000 µm, in particular in the range of about 700 to 1200 nm. An infrared absorbing compound may be incorporated into the drug delivery device according to the invention in several manners, as is disclosed in WO 2008/016297, incorporated by reference herein.

If a source of ultrasonic sound is used as energy source, it is preferred that the ultrasonic sound has a frequency of 2 x 10⁴ Hz to 1 x 10⁷ Hz (20 kHz to 10 MHz), more preferably 5 x 10⁵ Hz to 5 x 10⁶ Hz (500 kHz to 5 MHz). In order to protect the surrounding environment from the supply of energy by ultrasonic sound waves, it is preferable that the sound waves are focussed in a beam. It is also preferred that the ultrasound has an intensity of 0.1 to 20.0 W/cm², preferably 0.2 to 5.0 W/cm².

Experiments have further shown that it is preferred that the external energy source is used in such a way that the thermo-sensitive polymeric material is subjected to one or more, more preferably two or more, reversible glass transitions between a glassy state and a rubbery state. It has been found that such transitions, which as such require relatively little energy, can lead to very appreciable modifications in the transport characteristics, which, in turn, can lead to a very appreciable increase in the diffusion of an active compound present in the material. According to a particularly preferred application, the glass transition or glass transitions occur at a temperature of about 0 to about 90°C, more preferably in the range of about 30 to about 90°C, even more preferably in the range of about 35 to about 85°C, even more preferably in the range of about 40 to about 80°C, and most preferably in the range of about 45 to about 75°C.. Experiments show that these temperature ranges are adequate to obtain the particular advantages according to the present invention.

According to the preferred embodiments of the method for releasing an active compound from a drug delivery device according to the invention, the energy is supplied pulse-wise. Moreover, it is preferred that the energy is supplied from an energy source that is external with respect to the material. The advantages of these preferred embodiments are that the release of the active compound(s) can be controlled pulse-wise and that the energy can be supplied remotely from the patient by an intervention of the physician, non-academic hospital personnel such as nurses, or the patient himself, respectively. Depending on the nature of the complaint to be treated and the characteristics of the drug delivery device employed and the active compound present therein, the physician or the patient can regulate the duration, the frequency and the intensity of the release of the active compound. Preferably, the energy source is used in such a way that the material is subjected to one or more, preferably two or more, reversible glass transitions between a glassy state and a rubbery state.

### Examples

### Example 1: Synthesis of poly(styrene/n-butyl (meth)acrylate)

### Synthesis of poly(styreneln-butyl acrylate) PS-BA

The copolymer of styrene and n-butyl acrylate is prepared using a free radical polymerization procedure as described below. The two monomers are heated in the presence of azobisisobutyronitrile (AIBN) as a radical initiator. After the copolymerization, methanol is added to precipitate the polymer.

### Chemicals

- Inhibitor removers CAS 311332-500G (Aldrich 209326)
- n-Butylacrylat 99% CAS141-32-2 (Aldrich 23492-3)
- n-Butylacetate VWR 22081361 (0.88 g/cm³)
- Styrene 99% CAS 100-42-5 (Sigma 5497-2)
- AIBN
- Methanol
- Argon (g)

### Analysis

The copolymers were analysed with the following methods:
- Size exclusion chromatography to determine the weight average molecular weight (M_{w}), the number average molecular weight (Mₙ) and the polydispersity index (PDI)
- ¹H NMR spectroscopy to determine the monomer composition in the copolymer (ratio PS/BA)
- Differential scanning calorimetry to determine the T_{g}

**Table 1: Overview of several synthesized copolymersof styrene and n-butyl acrylate**

| | ***RD11007-1 PP2*** | ***RD11007-2 PP3*** | ***RD11007-C PP4*** | ***RD08006-C2 PP1*** |
|---|---|---|---|---|
| Styrene (S) | 200 g | 200 g | 168 g | 168 g |
| | 1.92 mol | 1.92 mol | 1.615 mol | 1.61 mol |
| n-butyl acrylate (BA) | 76.87 g | 105.1g | 51.85 g | 52 g |
| | 0.60 mol | 0.82 mol | 0.405 mol | 0.41 mol |
| S/BA (mol%) | 76/24 | 70/30 | 80 / 20 | 80 / 20 |
| AIBN (164.2 g/mol) | 0.83 g | 2.7 g | 3.58 g | 2.26 g |
| | 0.005 mol (0.2%) | 0.016 mol (3x 0.2%) | 0.022 mol (3x 0.36%) | 0.014 mol (0.7%) |
| Reaction time [min.] | 120 | 390 | 435 | 2400 |
| Ratio PS/BA (mol%) | 78/22 | 71/29 | 80/20 | 80/20 |
| T_{g}[°C] | 60 PTG | 48 | 61 | 61 |
| | 58 LSt | | | |
| M_{w} [kDa] | 73 | 66 | 23.1 | 37 |
| Mₙ [kDa] | 45 | 37 | 12.3 | 20 |
| PDI | 1.61 | 1.78 | 1.89 | 1.85 |
| Yield | 29 g | 97 g | 112.5 g | 134 g |
| | 10.5% | 32% | 56% | 61% |

### Example 1A: PS-BA PP2

Styrene and n-butyl acrylate were filtered over an inhibitor remover prior to use. A solution of styrene (200,00 g; 1,92 mol) and n-butyl acrylate (76,87 g; 0,60 mol) in n-butyl acetate (413 mL) was mechanically stirred in a 1L double-walled reactor under argon at *ca* 23°C. After degassing for 15 min. (∼10 argon/vacuum cycles), the mixture was heated to 70°C and a degassed solution of AIBN (0.83 g; 0.05 mol) in n-butyl acetate was added. The colorless solution was allowed to stir during - 2h. After cooling to ambient temperature, the mixture was poured into 2 L of methanol. The resulting polymer lump was separated from the supernatant by filtration on a glass frit, covered by methanol (0.5 L), stirred for 2h and filtered. The latter operation was repeated one more time. The final white precipitate was collected by filtration, washed twice with methanol and dried 24 h *in vacuo* at 60°C (Yield = 29 g, 10.5%).

### Analysis

- Tg: 60°C (PTG) / 58°C
- Ratio PS-BA: 78/22 (¹H NMR, CDCl₃)
- Mₙ: 45 kDa; M_{w}: 72 kDa: PDI = 1.61

### Example 1B: PS-BA 2 PP3

This copolymer was prepared according to the procedure described for **PS-BA 1** from a solution of styrene (200 g; 1.92 mol) and n-butyl acrylate (105.13 g; 0.82 mol) in butyl acetate (420 mL) at 69°C. A degassed suspension of AIBN (2.7 g; 0.016 mol) in butyl acetate (20 mL) was added in three times at intervals of ∼ 2h. The total reaction time was approximately 6.5 h. Half of the solution was poured into methanol (1 L). The resulting polymer lump was stirred during 5 minutes, separated from the supernatant by decantation and covered by methanol, into which the second fraction of the reaction mixture was subsequently poured. After five minutes, the lump was separated from methanol, covered with ethanol (500 mL) and stirred (20 minutes). The latter operation was repeated twice, upon 500 and 1500 mL ethanol addition, and stirring (overnight and 1h, respectively). Drying *in vacuo* was performed first at 23°C during 5 h, then overnight at 80°C, affording 105 g of a colorless material, which was dissolved in CHCl₃ (550 mL) and reprecipitated in MeOH (2 L) by dropwise addition. The polymer was then filtered, stirred in 1 L of methanol during 1 h (the latter operation was performed in total three times) and dried *in vacuo* for 96 h at 40°C (97.3 g; 32%).

### Analysis PP3 - after precipitation (for details see LB52, pages 53, 56, 59 and 62)

- 97.3 g
- Tg:48°C
- Ratio: PS-BA 71/29 (¹H NMR, CDCl₃)
- Mₙ: 36.9 kDa M_{w} = 65.8 kDa, PDI = 1.78

### Example 1C: PS-BA PP4

Styrene, n-butyl acrylate and AIBN were used as received, without any further purification. A solution of styrene (168.17 g; 1,615 mol) and n-butyl acrylate (51.85 g; 0,405 mol) in toluene (775 mL) was mechanically stirred in a 3-necked 2 L round bottom flask under argon at *ca* 23°C. After degassing for 15 min. (∼10 argon/vacuum cycles), the mixture was heated to 80°C (oil bath temperature) and AIBN (1.22 g; 7.44 mmol) was then added. The colorless solution was allowed to stir during - 2h15 before addition of an extra portion of AIBN (1.20 g; 7.32 mmol). After 2 more hours, a final batch of AIBN (1.16 g; 7.07 mmol) and added and the reaction stirred during 3 h. After cooling to ambient temperature, 400 mL of the mixture were poured into 2 L of methanol. The resulting polymer lump was separated from the supernatant by decantation and filtration on a filter paper. A similar procedure was followed for two more fractions of the mixture, of respectively 400 (methanol: 2 L) and 200 mL (MeOH: 1.5 L). The lumps/precipitate were combined and stirred overnight in methanol (1.5 L), affording a white solid along with a remaining sticky lump fraction. The precipitate was separated by filtration, the lump covered with methanol (1 L) and stirred until full transformation into a non-sticky solid, which was then filtered. The combined solids were washed with methanol (3 x 150 mL) and dried *in vacuo* at 70°C during 4 days. (122.5 g, 56 %).

### Analysis

- Tg: 61 °C
- Ratio PS-BA: 80/20 (1H NMR, CDCl₃)
- Mₙ: 12.3 kDa; M_{w}: 23.1 kDa: PDI = 1.89

### Synthesis of poly(styrene/n-butyl methacrylate) PS-BMA

The copolymers of styrene and n-butyl methacrylate, i.e. PS-BMA copolymers, are prepared using a free radical polymerization procedure. The two monomers (neat or in solution) are heated in the presence of azobisisobutyronitrile (AIBN) as a radical initiator. After the copolymerization methanol is added to the polymerization mixture, resulting in precipitation of the copolymer. The polymer is further purified by precipitation from chloroform or dichloromethane into methanol. Finally the product is dried in a vacuum oven.

### Example 1D: RH426:

Styrene and n-butyl methacrylate were filtered over aluminum oxide to remove the inhibitor prior to use. AIBN was recrystallized from methanol before use.

Styrene (58,9 g; 0,57 mol), n-butyl methacrylate (48.25 g; 0,34 mol) and AIBN (297 mg; 1,8 mmol) were placed in a roundbottom flask and bubbled 30 minutes with argon at ambient temperature. Subsequently, the roundbottom flask was placed in an oilbath preheated to 70°C. After 35 min stirring at 70°C, the polymerization mixture was poured into methanol (700 mL) resulting in the formation of a white precipitate, which was collected by filtration. The resulting solid was dissolved in chloroform (100 mL) and reprecipitated by dropwise addition to methanol (700 mL). The resulting solid was collected by filtration and dried under reduced vacuum at 40°C for 60 hours resulting in 4.7 gram of PS-BMA (5% isolated yield).

### Analysis

Copolymers were analyzed with the following methods:
- Size exclusion chromatography to determine the weight average molecular weight (M_{w}), the number average molecular weight (Mₙ) and the polydispersity index (PDI)
- ¹H NMR spectroscopy to determine the monomer composition in the copolymer (ratio PS/BMA)
- Differential scanning calorimetry to determine the T_{g}

**Table 2: Overview of several synthesized copolymersof styrene and n-butyl methacrylate.**

| | **RD08006-A1** | **RD08006-B2** | **RD08006-C1** | **RD08006-C2** |
|---|---|---|---|---|
| PS-BMA (mol%) | 64/36 | 71/29 | 78/22 | 80/20 |
| Tg (°C) | 51 | 56 | 51 | 61 |
| Mw (kDa) | 120 | 100 | 97 | 37 |
| Mn (kDa) | 80 | 62 | 62 | 20 |

### Example 2: Release experiments

### General procedure

An object comprising a thermo-sensitive polymer and an active comopund, prepared by compouding, having a size of approximately 2 cm and a thickness of approximately 2 mm was placed in a vessel containing 0.5 liter physiological buffer. The object consisted of a strand of a copolymer of styrene and n-butyl (meth)acrylate (PS-BA or PS-BMA), wherein an active compound was incorporated.

The vessel was first placed in a shaking bad of a temperature below the glass transition temperature T_{g} of the object (the "OFF switch"). The release rate of said active compound into the physiological buffer was determined by measuring the amount of active compound as a function of time. Then the vessel was placed in a shaking bath of a temperature above the T_{g} of the object (the "ON switch"), and again the release rate of said active compound into the physiological buffer was determined by measuring the amount of active compound as a function of time. This series of temperature cycles, measurements of active compound concentration and calculation of release rates was repeated over a period of time varying from 30 to 130 days.

The concentration of active compound in the physiological buffer was determined by several methods, e.g. Graphite Furnace Atomic Absorption Spectrometry (GF-AAS), UV-VIS, GC-MS, HPLC.

GF-AAS analyses were performed on an Analitik Jena AAS 5EA make model spectrometer equipped with an Analitik Jena, Broad-Range Photon Multiplier detector.

HPLC analyses were performed with a Shimadzu model 10ADvp: System controller SCL-10avp, Liquid chromatograph LC-10ADvp, Degasser DGU14A, Auto Injector SIL-10Advp, Column oven, Column Oven CTO10Avp/CTO10ACvp chromatograph, using a Shimadzu SPD-20A/SPD-10Advp detector.

### Example 2A: PS-BA 80/20 (PP1) with silver acetate

The release experiment as described above was performed using a copolymer of styrene and n-butyl acrylate in a ratio of 80 to 20 mol%, comprising 2 wt.% of silver acetate. The temperature during the "OFF switch" was 37.2°C (temperature of the human body), the temperature during the "ON switch" was 75°C. The object was kept at the temperature of the "ON switch" during a period of 60 minutes. The concentration of Ag⁺ was determined by Graphite Furnace Atomic Absorption Spectrometry (GF-AAS) at 328 nm.

The release rate during the "ON switch", i.e. the ON release, was determined to be 2,9 ng/min*cm², and the release rate during the "OFF switch", i.e. the OFF release rate, was determined to be 0,07 ng/min*cm². The ON release rate is calculated by taking the amount of silver ions released during the 15 minutes that is triggered. The OFF release rate is calculated over the period that the release is linear. It takes approximately 1 day from the 60 minutes trigger when this it achieved. The period in between is in this model neglected.

The release profile of the active compound as a function of time and temperature is shown in Figure 1 and Figure 2, wherein the concentration of silver ions is shown on the left-handed Y-axis, and the temperature on the right-handed Y-axis. Figure 1 and 2 show that during the "OFF switch", i.e. at a temperature below Tg, the amount of active compound in the physiological buffer does not increase significantly, indicating that release of active compound at a temperature below T_{g} of the PS-BA copolymer is negligible. During the "ON switch", i.e. at a temperature above Tg, the release of active compound is significant. Figure 1 and 2 also show that said release of active compound during the "On switch" can be repeated over a long period of time.

### Example 2B: PS-BA 80/20 (PP1) with mandelic acid

The release experiment as described above was performed using a copolymer of styrene and n-butyl acrylate in a ratio of 80 to 20 mol%, comprising 20 wt.% of mandelic acid. The temperature during the "OFF switch" was 25°C, the temperature during the "ON switch" was 70°C during the first 4 switches, and 75°C thereafter. The object was kept at the temperature of the "ON switch" during a period of 15 minutes. The concentration of mandelic acid in the physiological buffer was determined by HPLC (detection at 210 nm).

The release profile of the active compound as a function of time and temperature is shown in Figure 3, wherein the concentration of mandelic acid is shown on the left-handed Y-axis, and the temperature on the right-handed Y-axis. Figure 3 shows that during the "ON switch", in particular at a temperature of 75°C, the release of active compound is significant. Figure 3 also show that said release of active compound during the "On switch" can be repeated over a long period of time.

### Example 2C: PS-BA 71/29 (PP2) with ibuprofen

The release experiment as described above was performed using a copolymer of styrene and n-butyl acrylate in a ratio of 78 to 22 mol% (PP2), comprising 40 wt.% of inbuprofen. The temperature during the "OFF switch" was 37.2°C (temperature of the human body), the temperature during the "ON switch" was 70°C. The object was kept at the temperature of the "ON switch" during a period of 15 minutes. The concentration of ibuprofen in the physiological buffer was determined by HPLC, detection at 223 nm.

The release profile of the active compound as a function of time and temperature is shown in Figure 4, wherein the concentration of ibuprofen is shown on the left-handed Y-axis, and the temperature on the right-handed Y-axis. Figure 4 shows that during the "ON switch", the release of active compound is significant. Figure 4 also show that said release of active compound during the "On switch" can be repeated over a long period of time, although after the fourth cycle release during the "OFF switch", after about 650 hours, increased significantly.

### Example 2D: PS-BA 71/29 (PP3) with chlorhexidine

The release experiment as described above was performed using a copolymer of styrene and n-butyl acrylate in a ratio of 71 to 29 mol%, comprising 20 wt.% or 30 wt.% of chlorhexidin. The temperature during the "OFF switch" was 37.2°C (temperature of the human body), the temperature during the "ON switch" was 75°C. The object was kept at the temperature of the "ON switch" during a period of 60 minutes. The concentration of chlorhexidine in the physiological buffer was determined by HPLC or UV-VIS.

The release profile of chlorhexidin as a function of time and temperature is shown in Figure 5, wherein the concentration of chlorhexidin is shown on the left-handed Y-axis, and the temperature on the right-handed Y-axis. For the PS-BA comprising 20 wt.% of chlorhexidine, the ON release rates varied between 20 and 100 ng/(cm² *min). The release rate decreased after the third trigger. For the PS-BA comprising 30 wt.% of chlorhexidine, the ON release rates varied between 90 - 200 ng /(cm² *min). A significant release during the OFF switch is shown.

### Example 2E: PS-BA 80/20 (PP4) with chlorhexidine

The release experiment as described above was performed using a copolymer of styrene and n-butyl acrylate in a ratio of 80 to 20 mol% (PP4), comprising 20 wt.% of chlorhexidin diacetate. The temperature during the "OFF switch" was 22°C, the temperature during the "ON switch" was 75°C. The object was kept at the temperature of the "ON switch" during a period of 60 minutes. The concentration of chlorhexidine in the physiological buffer was determined by HPLC or UV-VIS.

The release profile of chlorhexidin as a function of time and temperature is shown in Figure 6, wherein the concentration of chlorhexidine is shown on the left-handed Y-axis, and the temperature on the right-handed Y-axis. Release of chlorhexidine was shown after 40 days. The ON release rates varied between 400 and 1200 ng/(cm² *min). The OFF release rates varied between 1 - 33 ng/(cm² *min).

### Example 2F: PS-BA 78/22 (PP2) with ibuprofen

The release experiment as described above was performed using a copolymer of styrene and n-butyl acrylate in a ratio of 78 to 22 mol% (PP1), comprising 40 wt.% of ibuprofen. The temperature during the "OFF switch" was 37.2°C, the temperature during the "ON switch" was 60°C for the first five pulses, and 70°C thereafter. The object was kept at the temperature of the "ON switch" during a period of 15 minutes. The concentration of ibuprofen in the physiological buffer was determined by HPLC.

Figure 7 shows that the copolymer PS-BA is able to release ibuprofen (IBU) pulse-wise over a period of at least 35 days. The release profile in Figure 7 shows 7 released pulses of IBU at 2 different trigger temperatures (see pink line).

The release rates are calculated at 37.2°C (OFF state) and during a pulse (ON state). These release rates are displayed in Figure 8.

### Example 2G: PS-BMA 64/36 with ibuprofen

The release experiment as described above was performed using a copolymer of styrene and n-butyl methacrylate in a ratio of 64 to 36 mol%, comprising 40 wt.% of ibuprofen. The temperature during the "OFF switch" was 37.2°C, the temperature during the "ON switch" was 65°C initially, and thereafter 70°C. The object was kept at the temperature of the "ON switch" during a period of 15 minutes. The concentration of ibuprofen was determined by HPLC with detection at 223 nm.

Figure 9 shows the release profile of ibuprofen as a function of time and temperature, wherein the concentration of ibuprofen is shown on the left-handed Y-axis, and the temperature on the right-handed Y-axis. Figure 9 shows that the copolymer PS-BMA is able to release ibuprofen (IBU) pulse-wise over a period of time.

The release rates are calculated at 37.2°C (OFF state) and during a pulse (ON state). These release rates are displayed in Figure 10.

### Example 3: Release of active compound directly from insulation materials

Tecothane TT-1095A was dissolved in THF, 10 wt% chlorhexidine was added. This mixture was then dried and part of this film was then used in a water bath release experiment as described above. The film was submerged in water and placed in a water bath at 37°C. Figure 11 shows that Tecothane TT-1095A showed direct release of Chlorhexidine without a delay. The experiment was repeated using Tecophilic HP60D10 and Tecoflex EG 93A, results are also shown in Figure 11.

### Example 4: Antimicrobial release measurements

### Bacterial culture preparation

S. aureus ATCC 49230 bacteria were cultured from cryopreservative beads (Protect Technical Surface Consultants Ltd., UK) onto blood agar plates at 37°C in ambient air. Consecutively several colonies were selected to inoculate 5 ml of tryptone soy broth (TSB, Oxoid, Basingstoke, Great Britain) at 37°C for 24 h in ambient air. This preculture was diluted 1:20 in 200 mL TSB and grown statically for 16 h at 37°C. Cultures were harvested by centrifugation (Beckman J2-MC centrifuge, Beckman Coulter, Inc. Brea, CA, USA) for 5 min at 5000 g in a JA14 rotor and the bacteria were resuspended in 10 mL PBS. Centrifugation was done twice in order to remove all traces of growth medium. Staphylococci were resuspended in PBS to a concentration of 2x10⁴ bacteria/ml.

### Sample Triggering

Droplets of 50 µl demineralized water were carefully positioned on the surface of disk-shaped samples of Poly(styrene/n-butylacrylate) Chlorhexidine gluconate with 20% and 30% Chex (PS/BAC20, PS/BAC30), Poly(styrene/n-butylacrylate) AgAc (BS/BAAg) and on control samples without antimicrobials (PS/BA). Prior to use, samples were rinsed by 3 minutes sonication in 70% ethanol followed by air drying.

A heating trigger was applied to each of these samples by putting the discs with the droplet into an incubator at 60°C during 1 h. The moisture level was kept high in order to limit evaporation. Consecutively the samples were cooled down to approximately 37°C and left at 37°C during 24 h during which the moisture level was kept high to limit evaporation.

### Antimicrobial assay

Each of the samples with the droplets on top was carefully transferred to the bottom sheet of a Petrifilm Aerobic Count plate (3M Microbiology, St. Paul, MN, USA) after which 5 µl of bacterial suspension (effectively 10³ bacteria) was added to the droplet to reach the desired bacterial challenge. Consecutively the Petrifilm system was closed in order to bring the entire volume of the drop, including the released antimicrobials and the bacteria, into close contact with the top sheet of the Petrifilm, which contains a transparent agar gel. The Petrifilm was incubated, together with the sample, for 48 h after which the number of colonies formed on the gel were counted, which appeared as red dots.

After CFU counting, the samples were thoroughly rinsed by 3 minutes sonication in 70% ethanol followed by air drying. Droplets of 50 µl demineralized water were carefully positioned on the surface of the samples again, on the same position as the previous droplet, incubated with 5 µl of bacterial suspension (effectively 10³ bacteria). Without a preliminary temperature triggering procedure, the samples with the bacterial challenges were transferred to a Petrifilm assay, consecutively incubated during 48 h after which CFU's were directly counted from the Petrifilm display.

This entire procedure, sample triggering and Petrifilm assay, was repeated four times with the same samples (see also Figure 12). The samples were kept in PBS buffer during two days prior to a next temperature trigger. Between trigger 3 and 4, the samples were kept in PBS during 6 weeks during continuous shaking. Each experiment was executed with three samples and with separately cultured bacteria.

In order to quantify the antimicrobial activity of the samples, a measure R is introduced as R=-log(C/A) with A the number of bacteria within the inoculum (1000) and C the number of bacteria on the samples after incubation. Because the Log function is not defined for the value zero, we interpret zero counts as C=0.1, otherwise we took the average over all measurements to be reflected in C. In addition we assume that on the control samples, for which the number was too high to count, after incubation 90% of the bacteria from the inoculation form colonies (thus taking C=900). This seems reasonable because in general 5-10% of the bacteria counted is dead and will not form colonies.

Figure 12 shows the procedure for the antimicrobial assays and antimicrobial activity assessment of antimicrobials released after a temperature trigger. After rinsing, the sample is exposed to 60°C during 1 h, during which antimicrobial release takes place towards a water droplet (Phase 1, triggered release). During an extended period of 24 h a decreasing amount of antimicrobials releases until the matrix is fully closed to inhibit further release (Phase 2: tail release). After that, the sample with the droplet is transferred to a Petrifilm™ bottom sheet, a bacterial challenge is injected into the droplet (including the released antimicrobials) and finally the Petrifilm™ is closed to inoculate the agar-gel on the transparent top sheet of the assay. After 48 h incubation at 37°C, during which no or only a limited amount of release is supposed to take place (Phase 3: shut down), the number of bacterial colonies is counted directly from the Petrifilm™. In order to evaluate the amount of released antimicrobials 3 days after triggering (Phase 4: continued shut down), the sample is rinsed and a water droplet with a bacterial challenge is positioned on the sample again, after which antimicrobial activity of the sample is tested during 48 h of incubation within the Petrifilm™. The efficacy of repeated triggers is tested by repeating the full procedure with the same sample several times.

Figure 13 shows the results of the measurement of the antimicrobial efficacy during two periods in time after the temperature trigger. The first measurement reflects the result of the active release of antimicrobials during 24 hours (light grey bars), as a result of the "open" polymer structure due to a temperature trigger beyond the Tg. The second measurement after 73 hours reflects the situation after returning to a temperature well below T_{g} during which the "closed" state of the polymer matrix is supposed to effectively inhibit antimicrobial release (dark grey bars). These measurements were repeated three times for each of the samples, by three independent triggers. Apparently the PS/BAAg samples were not active except 24h after the first trigger. The PS/BAC20 and PS/BAC30 samples, however, showed a high activity, sustained even after the third trigger, but with a non-zero activity after 72h, decreasing along with each trigger. Also the 24h activity appeared to decrease for the low concentration Chex samples (PS/BAC20) with repeating the triggers.

Control samples did not show any measurable activity, which means that colonies formed were too numerous to be counted (CFU>>200).

## Claims

1. A drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the characteristics of diffusion or transport of the active compound can be modified in a reversible manner by supplying energy from an energy source.

2. Drug delivery device according to claim 1, wherein the active compound is mixed with the thermo-sensitive polymeric material.

3. Drug delivery device according to claim 1 or claim 2, comprising particles comprising an active compound and an inert support material, wherein said particles are coated with a layer of said thermo-sensitive polymeric material.

4. Drug delivery device according to any one of the preceding claims, wherein the drug delivery device further comprises (c) a material being thermally insulating and permeable for the active compound.

5. Drug delivery device according to any one of the preceding claims, comprising:
(i) a central region comprising said active compound (a) and said thermo-sensitive polymeric material (b), and
(ii) an enveloping material (c) surrounding said central region, said enveloping material being thermally insulating and permeable for the active compound.

6. Drug delivery device according to claim 4 or claim 5, wherein the material being thermally insulating and permeable for the active compound (c) is a hydrogel.

7. Drug delivery device according to claim 4 or claim 5, wherein the material being thermally insulating and permeable for the active compound (c) is an aromatic polyether-based thermoplastic polyurethane.

8. A catheter, comprising a drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the characteristics of diffusion or transport of the active compound can be modified in a reversible manner by supplying energy from an energy source.

9. A catheter, comprising a heating means and a drug delivery device according to any one of the preceding claims, said drug delivery device comprising:
(i) a central region comprising (a) an active compound; and (b) a thermo-sensitive polymeric material comprising a copolymer of styrene and C₁-C₁₂ alkyl (meth)acrylate having a barrier effect against diffusion of an active compound and having a glass transition temperature within the range of about 0° to about 90°C; and
(ii) an enveloping material (c) surrounding said central region, said enveloping material being thermally insulating and permeable for the active compound,
wherein the characteristics of diffusion or transport of the active compound can be modified in a reversible manner by supplying energy from said heating means.

10. Catheter according to claim 8 or claim 9, wherein the active compound (a) comprises chlorhexidine or a source of chlorhexidine ions, mandelic acid and/or a source of silver ions.

11. Catheter according to any one of claims 8 - 10, wherein the heating means comprises a heating filament.

12. A method for releasing an active compound from a drug delivery device, the drug delivery device comprising:
(a) an active compound, and
(b) a thermo-sensitive polymeric material comprising a copolymer of styrene and C₁-C₁₂ alkyl (meth)acrylate, having a barrier effect against diffusion of an active compound and having a glass transition temperature within the range of about 0° to about 90°C,
wherein the active compound is released from the drug delivery device by reversibly modifying the transport characteristics of the thermo-sensitive polymeric material by supplying energy from an energy source.

13. Method according to claim 12, wherein the energy is supplied pulse-wise.

14. Method according to claim 12 or claim 13, wherein the energy is supplied from an energy source that is external with respect to the thermo-sensitive polymeric material.

15. Method according to any one of Claims 12 - 14, wherein the energy source is a source for electric energy, ultrasonic sound, infrared radiation or magnetic energy.
